# EUROPEAN PATENT SPECIFICATION

(11) **EP 4 245 365 B1**
(45) Date of publication and mention of the grant of the patent: **07.05.2025**
(21) Application number: 22162493.5
(22) Date of filing: 16.03.2022
(51) Int. Cl.: A61P 1/16, A61P 3/00, A61P 3/06, A61P 3/08, A61P 29/00, C07D 451/06, A61K 31/46

(54) **A FXR SMALL MOLECULE AGONIST, THE PREPARATION AND USE THEREOF**
EIN KLEINMOLEKÜLIGER FXR-AGONIST, DESSEN HERSTELLUNG UND VERWENDUNG
UNE PETITE MOLÉCULE AGONISTE DU RÉCEPTEUR FXR, SA PRÉPARATION ET SON UTILISATION

(43) Date of publication of application: 20.09.2023
(73) Proprietor: Cascade Pharmaceuticals, Inc., Lingang New Area (Shanghai) Pilot Free Trade Zone Pudong New Area Shanghai 201419 (CN)
(72) Inventor: Zhao, Yishuang, Shanghai 201419 (CN); Zhang, Zhenwei, Shanghai 201419 (CN); Wu, Guohui, Shanghai 201419 (CN); Yang, Shengsheng, Shanghai 201419 (CN)
(74) Representative: dompatent von Kreisler Selting Werner - Partnerschaft von Patent- und Rechtsanwälten mbB

(56) References cited:
- EP-A1- 3 730 491
- EP-A1- 3 919 489
- EP-A1- 3 957 640
- WO-A1-2012/087519
- WO-A1-2020/114307

## Description

### Technical Field

The present invention belongs to the field of medicine, and relates to the preparation and use of a class of non-steroidal compounds as FXR agonists. In particular, it relates to a class of organic small molecule compounds that can act as FXR agonists and an enantiomer, diastereomer, tautomer, racemate, hydrate, solvate, or a pharmaceutically acceptable salt thereof, its preparation method and its application in the preparation of a medicine for treating FXR-related diseases.

### Background

Farnesoid X receptor is a member of the nuclear receptor superfamily. It is a ligand-dependent nuclear transcription factor, which is mainly expressed in liver, intestine, kidney, bile duct and other systems. FXR is also known as bile acid receptor because it can be activated by endogenous ligand bile acid and participate in important links such as bile acid metabolism and cholesterol metabolism. FXR is directly involved in regulating the expression of more than 300 genes including physiological processes such as lipid metabolism, glucose metabolism, inflammation, fibrosis, liver regeneration, cell differentiation and proliferation. In the natural environment, ligands of FXR include primary bile acid: chenodeoxycholic acid, secondary bile acid: lithocholic acid, deoxycholic acid, etc. For example, FXR, activated by endogenous ligand bile acids, plays an important role in triglyceride (TG) metabolism. FXR can regulate key enzymes, lipoproteins and corresponding receptors involved in the metabolism of TG, so that the content of TG in the liver and circulating blood can reach stable equilibrium. Therefore, up to now, several FXR synthetic ligand molecules have been applied in the field of metabolic diseases such as the liver.

FXR agonist molecules have shown excellent clinical effects in the treatment of liver diseases such as primary biliary cirrhosis (PBC), primary sclerosing cholangitis (PSC), nonalcoholic steatohepatitis (NASH) and so on. So far, the FXR agonist molecule obeticholic acid (OCA), which will be the first to be approved for marketing, has been proven to significantly improve a variety of metabolic symptoms, such as reducing liver fat content, reducing inflammation and inhibiting liver fibrosis. However, many clinical shortcomings of OCA have also been increasingly highlighted, such as causing itching, lowering high-density lipoprotein (HDLc), increasing low-density lipoprotein (LDLc) and so on. Therefore, in terms of clinical needs, new FXR agonist molecules with good clinical effects and low toxicity and side effects are urgently needed.

In addition, studies have confirmed that FXR is closely related to the occurrence and development of tumors. In a variety of tumors, FXR acts as a tumor suppressor gene. For example, in hepatocellular carcinoma and rectal cancer, the expression of FXR is low; and after the activation of FXR, the progression of liver or rectal cancer is significantly inhibited by inhibiting the activity of β-catenin. Recent studies have shown that in cholangiocarcinoma, FXR agonist OCA can significantly inhibit the proliferation, migration and colony formation of intrahepatic cholangiocarcinoma.

Furthermore, FXR agonists can be used as a new antiviral drug candidate, and studies have confirmed that FXR ligands can be used as a new therapeutic strategy for inhibiting the replication of hepatitis B virus (HBV). FXR agonists can inhibit HBV surface antigen synthesis, inhibit HBV DNA and RNA replication, and most importantly, inhibit HBV cccDNA production. In the aspect of hepatitis C virus (HCV), the FXR agonist GW4064 can inhibit the invasion of HCV into liver tissue cells by indirect means. Therefore, agonist molecules of FXR also have great prospects as antiviral drugs. FXR agonists are disclosed, for example in EP3957640, EP3919489, EP3730491 and elsewhere.

In summary, there is still a lack of novel FXR agonist molecules, which can be prepared by simple methods and have good inhibitory effects in the art.

### Summary of the Invention

The purpose of the present invention is to provide a novel FXR agonist molecule, which can be prepared by a simple method and have good inhibitory effects.

In the first aspect of the present invention, provided herein is a compound of formula I: 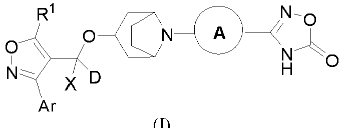 or an enantiomer, diastereomer, tautomer, racemate, hydrate, solvate, or pharmaceutically acceptable salt thereof;
wherein:
Ar is selected from the group consisting of a substituted or unsubstituted C₆-C₁₀ aryl, and substituted or unsubstituted 5-9 membered heteroaryl ring (including a single ring or fused ring containing 1-3 heteroatoms selected from O, S or N);
A is selected from the group consisting of a substituted or unsubstituted C₆-C₁₀ aryl, substituted or unsubstituted 5-9 membered heteroaryl ring (including a single ring or fused ring containing 1-3 heteroatoms selected from O, S or N);
R¹ is selected from the group consisting of a substituted or unsubstituted C₁-C₆ alkyl, substituted or unsubstituted C₃-C₆ cycloalkyl, and substituted or unsubstituted 5-9 membered heterocyclic group (containing 1-3 heteroatoms selected from O, S or N);
X is selected from the group consisting of H and D;
wherein, the "substituted" means that one or more hydrogen atoms on a group are each independently replaced by a substituent selected from the group consisting of a halogen, halogenated C₁-C₆ alkyl, halogenated C₁-C₆ alkoxy, C₁-C₆ alkyl, C₁-C₆ alkoxy, C₃-C₆ cycloalkyl, C₃-C₆ cycloalkoxy, cyano and nitro.

In another embodiment, the R¹ is selected from the group consisting of a substituted or unsubstituted C₁-C₆ alkyl, and substituted or unsubstituted C₃-C₆ cycloalkyl;
wherein the "substituted" means that one or more hydrogen atoms on a group are each independently replaced by a substituent selected from the group consisting of a halogen, halogenated C₁-C₆ alkyl, halogenated C₁-C₆ alkoxy, C₁-C₆ alkyl, C₁-C₆ alkoxy, C₃-C₆ cycloalkyl, C₃-C₆ cycloalkoxy, cyano and nitro.

In another embodiment, the Ar is selected from the group consisting of a substituted or unsubstituted C₆-C₁₀ aryl, substituted or unsubstituted 5-9 membered heteroaryl ring; and, the substituent is selected from the group consisting of H, F, Cl, Br, methyl, ethyl, n-propyl, isopropyl, n-butyl, isobutyl, trifluoromethyl, and trifluoromethoxy.

In another embodiment, the A is selected from the group consisting of a substituted or unsubstituted C₆-C₁₀ aryl, substituted or unsubstituted 5-9 membered heteroaryl ring; wherein, the substituent on the aryl or heteroaryl is selected from the group consisting of H, F, Cl, Br, methyl, ethyl, n-propyl, isopropyl, n-butyl, isobutyl, trifluoromethyl, and trifluoromethoxy.

In another embodiment, the Ar is selected from the group consisting of a substituted or unsubstituted phenyl, substituted or unsubstituted 5-7 membered heteroaryl ring (including a single ring or fused ring containing 1-3 heteroatoms selected from O, S or N).

In another embodiment, the A is selected from the group consisting of a substituted or unsubstituted phenyl, substituted or unsubstituted 5-7 membered heteroaryl ring (including a single ring or fused ring containing 1-3 heteroatoms selected from O, S or N).

In another embodiment, the A is a benzothiazole.

In another embodiment, the Ar or A are each independently selected from the group consisting of a substituted or unsubstituted phenyl, substituted or unsubstituted pyridyl, substituted or unsubstituted pyrimidinyl, substituted or unsubstituted pyridazinyl, substituted or unsubstituted pyrimidinyl, substituted or unsubstituted pyridazinyl, substituted or unsubstituted furyl, substituted or unsubstituted thienyl, substituted or unsubstituted pyrrolyl, substituted or unsubstituted thiazolyl, and substituted or unsubstituted imidazolyl.

In another embodiment, the R¹ is selected from the group consisting of a substituted or unsubstituted C₁-C₄ alkyl, and substituted or unsubstituted cyclopropyl.

In another embodiment, the Ar is a substituted or unsubstituted phenyl.

In another embodiment, the Ar is selected from the group consisting of a 2,5-dichlorophenyl, 2-methylphenyl, 2-trifluoromethylphenyl, 2-trifluoromethoxyphenyl.

In another embodiment, the R¹ is selected from the group consisting of a methyl, ethyl, n-propyl, isopropyl, n-butyl, isobutyl, cyclopropyl, cyclobutyl and cyclopentyl.

In another embodiment, the compound of formula I has the structure as shown in the formula below:

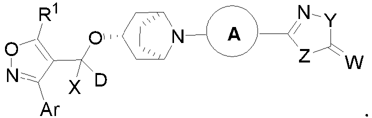

In another embodiment, the compound of formula I has the structure as shown in the formula below:

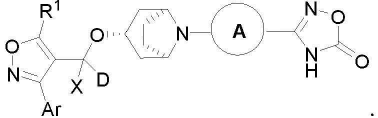

In another embodiment, the compound is selected from the group consisting of

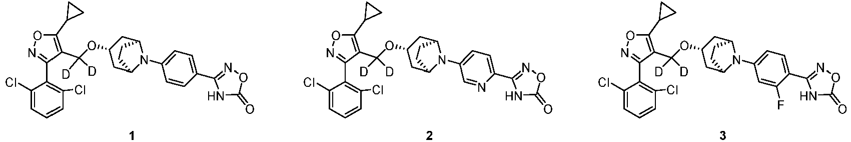

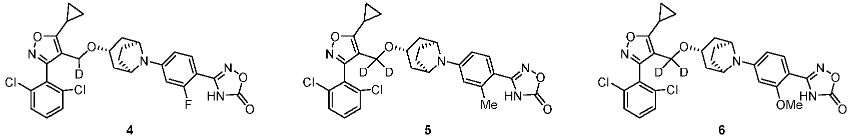

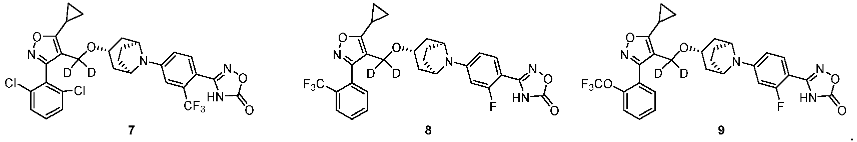

In the second aspect of the present invention, provided herein is a preparation method of the compound of the first aspect, and said method comprises preparing a compound of formula I by a method selected from the following route 1 or 2: 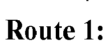 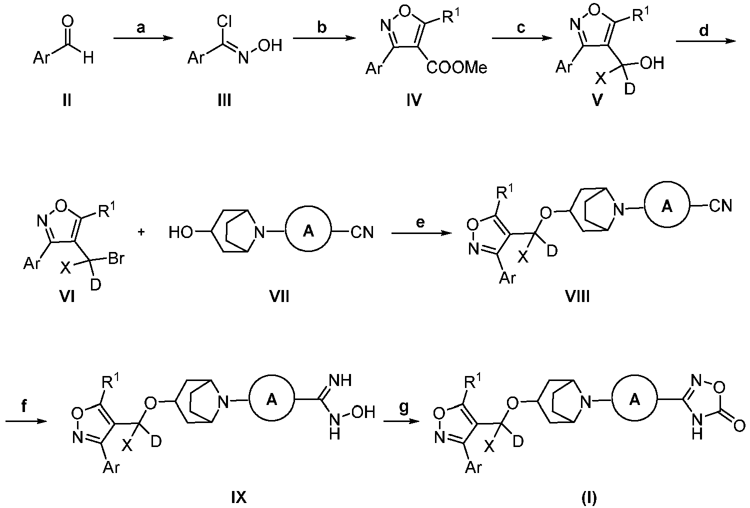
(a) a substituted benzaldehyde of formula **II,** as starting material, is reacted with hydroxylamine hydrochloride under the presence of a base to obtain the intermediate; the intermediate is then chlorinated by N-chlorosuccinimide (NCS) to form a compound of general formula **III;**
(b) the compound of formula **III** is then reacted with the corresponding 3-oxopropionate under the presence of a base to obtain a compound of formula **IV;**
(c) the ester compound of formula **IV** is reduced to the corresponding alcohol, i.e. a compound of formula **V,** under the presence of a deuterated reducing agent;
(d) the compound of formula **V** is brominated by bromine reagent to obtain a compound of **VI;**
(e) the compound of formula **VI** and the compound of formula **VII** are reacted under the presence of a base to obtain a compound of formula **VIII;**
(f) the compound of formula **VIII** is reacted with hydroxylamine hydrochloride under the presence of a base to obtain a compound of formula **IX;**
(g) the compound of formula **IX** is reacted under the presence of a phosgene, triphosgene or carbonyldiimidazole to obtain the compound of formula **I;**
   wherein X is a deuterium; and
   R¹, Ar, A are determined as described in the first aspect of the present invention; 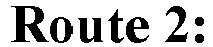 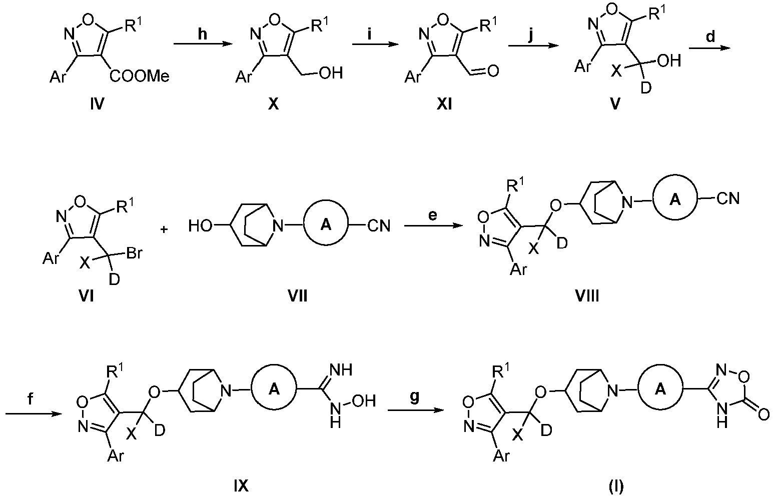
      (a) an ester compound of formula **IV** is reduced to the corresponding alcohol, i.e. a compound of formula **X,** under the presence of a reducing agent;
      (b) the compound of formula **X** is oxidized to the corresponding aldehyde, i.e. a compound of formula **XI,** under the action of an oxidizing agent;
      (c) the ester compound of formula **XI** is reduced to a compound of formula V under the presence of a deuterated reducing agent;
      (d) the compound of formula **V** is brominated by bromine reagent to obtain a compound of **VI;**
      (e) the compound of formula **VI** and a compound of formula **VII** are reacted under the presence of a base to obtain a compound of formula **VIII;**
      (f) the compound of formula **VIII** is reacted with hydroxylamine hydrochloride under the presence of a base to obtain a compound of formula **IX;**
      (g) the compound of formula **IX** is reacted under the presence of a phosgene, triphosgene or carbonyldiimidazole to obtain the compound of formula **I;**
         wherein X is a hydrogen; and
         R¹, Ar, A are determined as described in the first aspect of the present invention.

In another embodiment, the compound of formula **VII** is prepared through the step below: 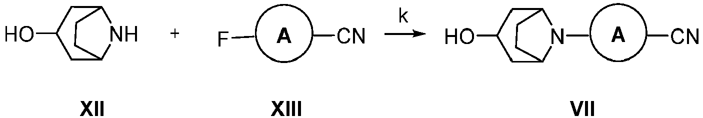 k) a compound of formula **XII** and a compound of formula **XIII** generate the compound shown in the general formula VII under the presence of a base;

In each formula, the definition of A is as described in the first aspect of the present invention.

In another preferred embodiment, when the product has an optical isomer, a raw material with the corresponding optical configuration is used for preparation.

In the third aspect of the present invention, provided herein is a pharmaceutical composition comprising the compound of formula **I,** or an enantiomer, diastereomer, tautomer, racemate, hydrate, solvate, or pharmaceutically acceptable salt thereof; and a pharmaceutically acceptable carrier.

In the fourth aspect of the present invention, provided herein is the use of the compound of formula **I,** or an enantiomer, diastereomer, tautomer, racemate, hydrate, solvate, or pharmaceutically acceptable salt thereof for the preparation of a pharmaceutical composition for treating diseases or conditions related to FXR activity or expression.

In another embodiment, the diseases related to FXR is selected from the group consisting of diseases related to bile acid metabolism, glucose metabolism, lipid metabolism, inflammation, and/or liver fibrosis process.

In another embodiment, the diseases related to FXR is nonalcoholic steatohepatitis (NASH), primary biliary cirrhosis (PBC), primary sclerosing cholangitis (PSC), gallstones, nonalcoholic cirrhosis, hepatitis B (HBV), hepatitis C (HCV), liver fibrosis, cholestatic liver disease, hyperlipidemia, hypercholesterolemia, or diabete.

In another embodiment, the pharmaceutical composition is used as an FXR agonist.

In another embodiment, the pharmaceutical composition is used to reduce the levels of ALP, ALT, AST and TBA in serum.

In another embodiment, the pharmaceutical composition is used to reduce the content of hydroxyproline in liver tissue.

In another embodiment, the pharmaceutical composition is used to downregulate the expression of α-SMA and Col1α1 mRNA in liver tissue.

In another embodiment, the pharmaceutical composition is used to inhibit the synthesis of HBV surface antigen, inhibit the replication of HBV DNA and RNA, and inhibit the production of HBV cccDNA.

In another embodiment, the pharmaceutical composition is used to reduce the collagen content in liver.

In another embodiment, the pharmaceutical composition is prepared by the following method: mixing the compound of formula **I** with pharmaceutically acceptable excipients (such as excipients, diluents, etc.), and formulating it into tablets, capsules, granules or syrup, etc.

It should be understood that within the scope of the present invention, the technical features mentioned above of the present invention and the technical features specifically described below, e.g., in the examples, can be combined with each other to form new or preferred technical solutions. Due to the limited pages, they are not described here. The scope of the present invention is defined in the appended claims.

### Detailed Description of the Invention

After extensive and deep research, the inventors of the present application have developed a class of non-steroidal compounds that can be used as FXR agonists and have agonistic ability to FXR at both molecular and cellular levels. Studies have shown that the compounds of the present application can reduce the levels of ALP, ALT, AST and TBA in serum, reduce the content of hydroxyproline in liver tissue, down-regulate the expression of α-SMA and Col1á1 mRNA in liver tissue, reduce the content of collagen in liver, inhibit the synthesis of HBV surface antigen, inhibit the replication of HBV DNA and RNA, and inhibit the production of HBV cccDNA. The compound of the present invention has advantages, such as high FXR agonistic activity, convenience in synthesis, easy availability of raw materials. The compound of the present invention can be used for preparing medicines for treating FXR-related diseases. On this basis, the present invention has been completed.

### Terms

In the present invention, unless otherwise indicated, the terms used herein have the ordinary meanings known to those skilled in the art.

In the present invention, the halogen is F, Cl, Br or I.

In the present invention, the term "C₁-C₆" means that a group has 1, 2, 3, 4, 5 or 6 carbon atoms, and "C₃-C₆" means that a group has 3, 4, 5 or 6 carbon atoms, and so on.

In the present invention, the term "alkyl" refers to a saturated linear or branched hydrocarbon moiety. For example, the term "C1-C6 alkyl" refers to a straight or branched chain alkyl group having 1 to 6 carbon atoms including, but not limited to, methyl, ethyl, propyl, isopropyl, butyl, isobutyl, sec-butyl, tert-butyl, pentyl, hexyl, etc.; preferably ethyl, propyl, isopropyl , butyl, isobutyl, sec-butyl and tert-butyl.

In the present invention, the term "alkoxy" denotes a -O-(C1-C6 alkyl) group. For example, the term "C1-C6 alkoxy" refers to a straight or branched chain alkoxy group having 1 to 6 carbon atoms, including, but not limited to, methoxy, ethoxy, propoxy, isopropoxy and butoxy, etc.

In the present invention, the term "cycloalkyl" refers to a saturated cyclic hydrocarbon moiety. For example, the term "C3-C6 cycloalkyl" refers to a cyclic alkyl group having 3 to 6 carbon atoms in the ring, including, but not limited to, cyclopropyl, cyclobutyl, cyclopentyl, cyclohexyl, etc.

In the present invention, the term "cycloalkoxy" means cycloalkyl-O-, wherein the cycloalkyl is defined as described above.

In the present invention, the term "aryl" refers to a hydrocarbyl moiety comprising one or more aromatic rings. Examples of aryl groups include, but not limited to, phenyl (Ph), naphthyl, pyrenyl, fluorenyl, anthracenyl, and phenanthryl.

In the present invention, the term "heteroaryl" refers to a moiety comprising one or more aromatic rings having at least one heteroatom, e.g. N, O or S. Examples of heteroaryl groups include furyl, pyrrolyl, thienyl, oxazolyl, imidazolyl, thiazolyl, pyridyl, pyrimidinyl, quinazolinyl, quinolinyl, isoquinolinyl, indolyl, and the like.

Unless otherwise indicated, alkyl, alkoxy, cycloalkyl, cycloalkoxy, aryl, and heteroaryl groups described herein are substituted and unsubstituted groups. Possible substituents on alkyl, alkoxy, cycloalkyl, cycloalkoxy, aryl and heteroaryl include, but not limited to: hydroxy, amino, nitro, nitrile, halogen, C₁-C₆ alkyl , C₂-C₁₀ alkenyl, C₂-C₁₀ alkynyl, C₃-C₂₀ cycloalkyl, C₃-C₂₀ cycloalkenyl, C₁-C₂₀ heterocycloalkyl, C₁-C₂₀ heterocycloalkenyl, C₁-C₆ alkoxy, Aryl, heteroaryl, heteroaryloxy, C₁-C₁₀ alkylamino, C₁-C₂₀ dialkylamino, arylamino, diarylamino, C₁-C₁₀ alkylsulfamoyl, arylsulfamoyl , C₁-C₁₀ alkylimino, C₁-C₁₀ alkylsulfoimino, arylsulfoimino, mercapto, C₁-C₁₀ alkylthio, C₁-C₁₀ alkylsulfonyl, arylsulfonyl, acylamino , aminoacyl, aminothioacyl, guanidino, ureido, cyano, acyl, thioacyl, acyloxy, carboxyl and carboxylate groups. On the other hand, a cycloalkyl group, heterocycloalkyl group, heterocycloalkenyl group, aryl group and heteroaryl group can also be fused with each other.

In the present invention, the substitution is monosubstitution or polysubstitution, and the polysubstitution is disubstitution, trisubstitution, tetrasubstitution, or pentasubstitution. The disubstitution refers to a group having two substituents, and so on.

The pharmaceutically acceptable salts of the present invention may be salts fromed from anions with positively charged groups on the compounds of formula I. Suitable anions are chloride, bromide, iodide, sulfate, nitrate, phosphate, citrate, methane sulfonate, trifluoroacetate, acetate, malate, tosylate, tartrate, fumarate, glutamate, glucuronate, lactate, glutarate or maleate. Similarly, salts can be formed from cations with negatively charged groups on compounds of formula I. Suitable cations include sodium, potassium, magnesium, calcium, and ammonium, such as tetramethylammonium.

In another preferred embodiment, the "pharmaceutically acceptable salt" refers to the salts formed from the compound of formula **I** with an acid selected from the group consisting of hydrofluoric acid, hydrochloric acid, hydrobromic acid, phosphoric acid, acetic acid, oxalic acid, sulfuric acid, nitric acid, methanesulfonic acid, sulfamic acid, salicylic acid, trifluoromethanesulfonic acid, naphthalenesulfonic acid, maleic acid, citric acid, acetic acid, lactic acid, tartaric acid, succinic acid, oxalic acid, pyruvic acid, malic acid, glutamic acid, p-toluenesulfonic acid, naphthalenesulfonic acid, ethanesulfonic acid, naphthalenedisulfonic acid, malonic acid, fumaric acid, propylene acid, oxalic acid, trifluoroacetic acid, stearic acid, pamoic acid, hydroxymaleic acid, phenylacetic acid, benzoic acid, glutamic acid, ascorbic acid, p-aminobenzenesulfonic acid, 2-acetoxybenzoic acid, and isethionic acid acid, etc.; or a sodium, potassium, calcium, aluminium or ammonium salt formed from a compound of formula I with an inorganic base; or the methylamine salt, ethylamine salt or ethanolamine salt formed from the compound of general formula I and an organic base.

In another preferred embodiment, in the compound, any one of ring A, Ar, X and R¹ is the corresponding group in the specific compound described in the embodiments.

The compounds of the present invention possess asymmetric centers, chiral axes and chiral planes, and may exist as racemates, R-isomers or S-isomers. Those skilled in the art can obtain the R-isomer and/or S-isomer from the racemate by conventional technical means.

### Method of preparation

The preparation method of the compound of formula **I** of the present invention is shown in the synthetic route below: 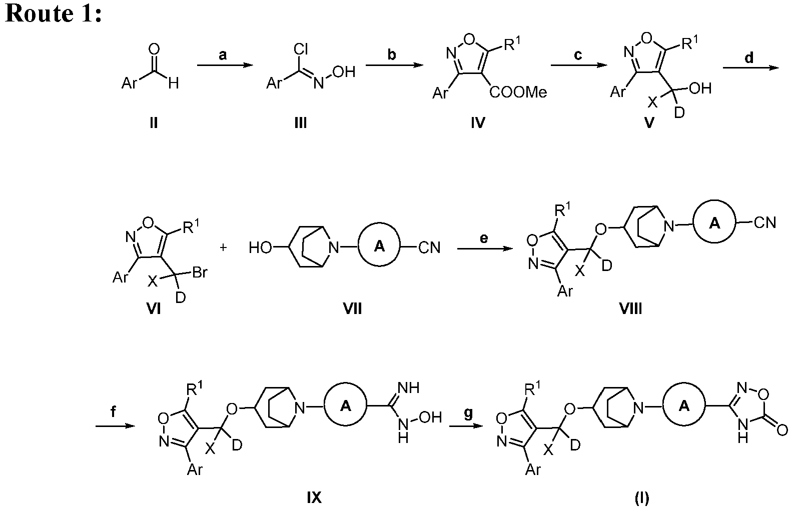
(a) a substituted benzaldehyde of formula **II,** as starting material, is reacted with hydroxylamine hydrochloride under the presence of a base to obtain the intermediate; the intermediate is then chlorinated by N-chlorosuccinimide (NCS) to form a compound of general formula **III;**
(b) the compound of formula **III** is then reacted with the corresponding 3-oxopropionate under the presence of a base to obtain a compound of formula **IV;**
(c) the ester compound of formula **IV** is reduced to the corresponding alcohol, i.e. a compound of formula **V,** under the presence of a deuterated reducing agent;
(d) the compound of formula **V** is brominated by bromine reagent to obtain a compound of **VI;**
(e) the compound of formula **VI** and a compound of formula **VII** are reacted under the presence of a base to obtain a compound of formula **VIII;**
(f) the compound of formula **VIII** are reacted with hydroxylamine hydrochloride under the presence of a base to obtain a compound of formula **IX;**
(g) the compound of formula **IX** is reacted under the presence of a phosgene, triphosgene or carbonyldiimidazole to obtain the compound of formula **I;**
   wherein X is deuterium; and
   R¹, Ar, A are determined as described above; 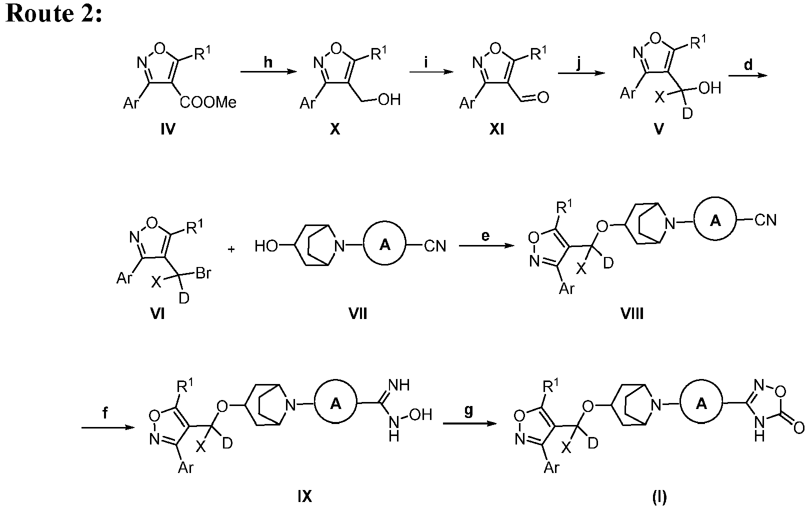
      (a) an ester compound of formula **IV** is reduced to the corresponding alcohol, i.e. a compound of formula **X,** under the presence of a reducing agent;
      (b) the compound of formula **X** is oxidized to the corresponding aldehyde, i.e. a compound of formula **XI,** under the action of an oxidizing agent;
      (c) the ester compound of formula **XI** is reduced to a compound of formula V under the presence of a deuterated reducing agent;
      (d) the compound of formula **V** is brominated by bromine reagent to obtain a compound of **VI;**
      (e) the compound of formula **VI** and a compound of formula **VII** are reacted under the presence of a base to obtain a compound of formula **VIII;**
      (f) the compound of formula **VIII** are reacted with hydroxylamine hydrochloride under the presence of a base to obtain a compound of formula **IX;**
      (g) the compound of formula **IX** is reacted under the presence of a phosgene, triphosgene or carbonyldiimidazole to obtain the compound of formula **I;**
         wherein X is hydrogen; and
         R¹, Ar, A are determined as described above.

In another embodiment, the compound of formula **VII** is prepared through the step below: 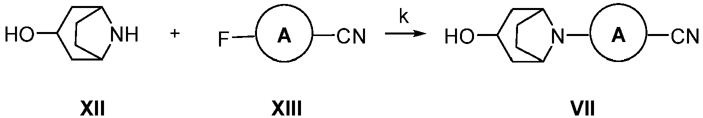 k) a compound of formula **XII** and a compound of formula **XIII** generate a compound shown in the general formula VII under the presence of a base;

In each formula, the definition of A is as described in the first aspect of the present invention.

### Pharmaceutical compositions and therapeutic uses thereof

The compounds provided herein can be used alone or mixed with pharmaceutically acceptable excipients, such as excipients, diluents, etc., to prepare tablets, capsules, granules or syrups for oral administration. The pharmaceutical composition can be prepared according to conventional methods in pharmacy. The pharmaceutical composition of the present invention contains the active ingredient in a safe and effective amount, and a pharmaceutically acceptable carrier.

The "active ingredient" in the present invention refers to the compound of formula I in the present invention.

The "active ingredients" and pharmaceutical compositions of the present invention are used to prepare medicines for treating FXR-related diseases.

The "active ingredients" and pharmaceutical compositions of the present invention are useful as FXR agonists. In another preferred embodiment, the active ingredient can be used to prepare a medicament for preventing and/or treating diseases regulated by FXR agonists.

The "safe and effective amount" refers to an amount of the active ingredient sufficient to significantly improve the condition without causing serious side effects. Typically, the pharmaceutical composition contains 1-2000 mg of active ingredient/agent, more preferably 10-200 mg of active ingredient/agent. Preferably, the "one dose" is one tablet.

"Pharmaceutically acceptable carrier" refers to one or more compatible solid or liquid filler or gel substances which are suitable for human use and must be of sufficient purity and sufficiently low toxicity. "Compatibility" as used herein refers to that the components of the composition can be blended with the active ingredients of the present invention and with each other without significantly reducing the efficacy of the active ingredients. Examples of pharmaceutically acceptable carrier moieties include cellulose and derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), moisturizing agents (such as sodium lauryl sulfate), colorants, flavors, stabilizers, antioxidants, preservatives, pyrogen-free water, etc.

The method of administration of the active ingredient or pharmaceutical composition of the present invention is not particularly limited. Representative methods of administration include (but not limited to): oral, intratumoral, rectal, parenteral (intravenous, intramuscular or subcutaneous) administration and the like.

Solid dosage forms for oral administration include capsules, tablets, pills, powders and granules.

Liquid dosage forms for oral administration include pharmaceutically acceptable emulsions, solutions, suspensions, syrups or tinctures. In addition to the active ingredient, liquid dosage forms may contain inert diluents conventionally employed in the art, such as water or other solvents, solubilizers and emulsifiers, for example, ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or mixtures thereof, and the like. Besides these inert diluents, the compositions can also contain adjuvants such as wetting agents, emulsifying and suspending agents, sweetening agents, flavoring agents and perfuming agents.

In addition to the active ingredient, suspensions may contain suspending agents such as ethoxylated isostearyl alcohols, polyoxyethylene sorbitol and sorbitan esters, microcrystalline cellulose, aluminum methoxide and agar, or mixtures thereof, and the like.

Compositions for parenteral injection may comprise physiologically acceptable sterile aqueous or anhydrous solutions, dispersions, suspensions or emulsions, and sterile powders for reconstitution into sterile injectable solutions or dispersions. Suitable aqueous and non-aqueous carriers, diluents, solvents or excipients include water, ethanol, polyols and suitable mixtures thereof.

The compounds of the present invention may be administered alone or in combination with other therapeutic agents, e.g., hypolipidemic agents.

When a pharmaceutical composition is used, a safe and effective amount of a compound of the present invention is administered in a mammal (e.g., a human) in need thereof, wherein the administered dose is a pharmaceutically effective dose. For a person weighing 60 kg, the daily dose is usually 1 to 2000 mg, preferably 20 to 500 mg. Of course, the route of administration, the patient's health and other factors should also be taken into account for a specific dosage, all of which are within the skill of the skilled physician.

The present invention will be further described below in conjunction with specific embodiments. It should be understood that these examples are only used to illustrate the present invention and not to limit the scope of the present invention.

In the following examples, the experimental methods without specific conditions are usually in accordance with conventional conditions, such as the conditions described in Molecular Cloning: A Laboratory Manual, Sambrook, etc, New York: Cold Spring Harbor Laboratory Press, 1989*,* or in accordance with the conditions suggested by the manufacturer. Unless otherwise indicated, percentages and parts are weight percentages and parts.

Unless otherwise indicated, all professional and scientific terms used herein have the same meanings as those familiar to those skilled in the art. In addition, any methods and materials similar or equivalent to those described can be used in the methods of the present invention. Preferred methods and materials described herein are provided for illustrative purposes only.

The instruments and main experimental materials used are as follows:
The used reagents and anhydrous solvents were purchased from Chinese commercial companies, and were directly used unless otherwise indicated. ¹H and ¹³C NMR were performed on Bruker AM-400 and Varian Mercury plus-400 nuclear magnetic resonance apparatus. Mass spectrometry was performed on Agilent 6230 mass spectrometer. 200-300 mesh column chromatography silica gel from Qingdao Ocean Chemical Plant and HSGF254 TLC plate from Yantai Chemical Research Institute were used.

The compound of the present invention was prepared according to any method selected from the following route 1 or 2, using suitable starting materials:

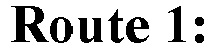

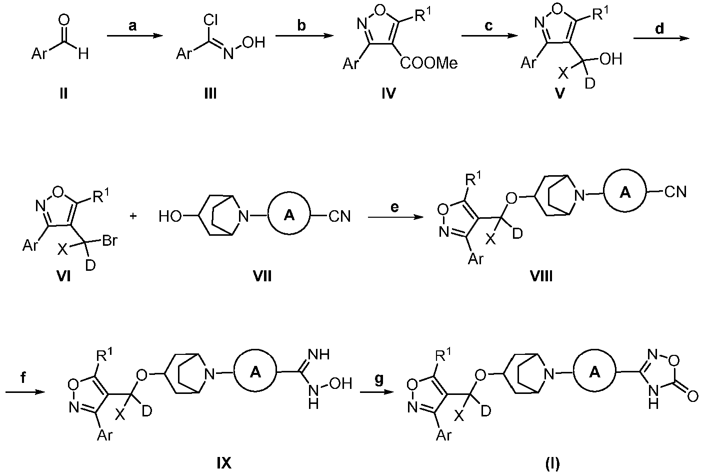

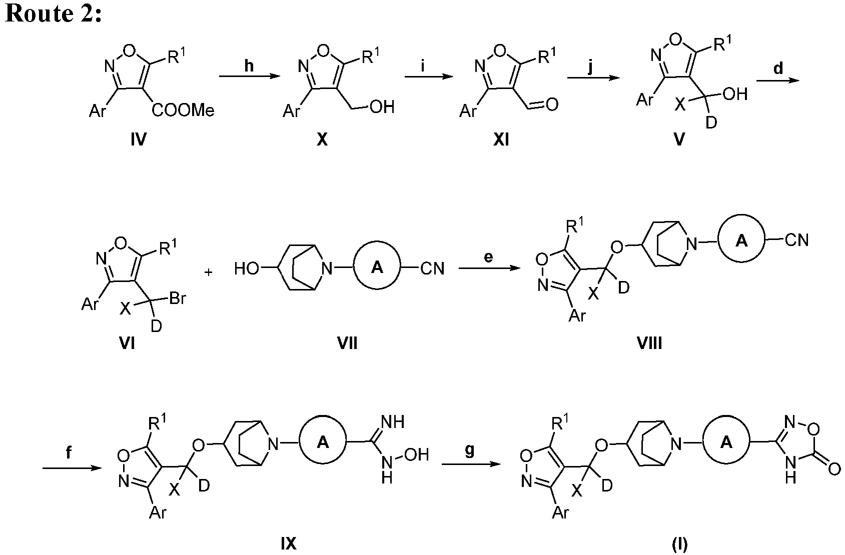

### Synthesis of Intermediate VII-1:

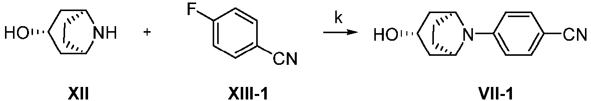

Endo-8-azabicyclo[3.2.1]octan-3-ol **XII** (10g, 78.7mmol) and p-fluorobenzonitrile **.XIII-1** (78.7mmol) were dissolved in N,N-dimethylformamide (150 mL). Potassium carbonate (197mmol) was added in batches at room temperature. The reaction was carried out at 80°C overnight. The reaction solution was diluted by adding ethyl acetate (500 mL), and washed with water. The aqueous phase was extracted with ethyl acetate (300 mL x 3 times). The organic phases were combined, washed with saturated brine, and concentrated. Intermediate **VII-1** (11 g, yield 61%) was obtained by column chromatography. ¹H NMR (400 MHz, DMSO-d₆) δ 7.51 - 7.48 (m, 2H), 6.82 - 6.79 (m, 2H), 4.62 (s, 1H), 4.27 (*d, J* = 5.2 Hz, 2H), 3.78 (s, 1H), 2.28 (d, *J* = 6.8 Hz, 2H), 1.91 - 1.85 (m, 4H), 1.57 (d, *J* = 14.0 Hz, 2H). MS(ESI, m/z): 229[M+H]⁺.

### Example 1, Synthesis of Compound 1:

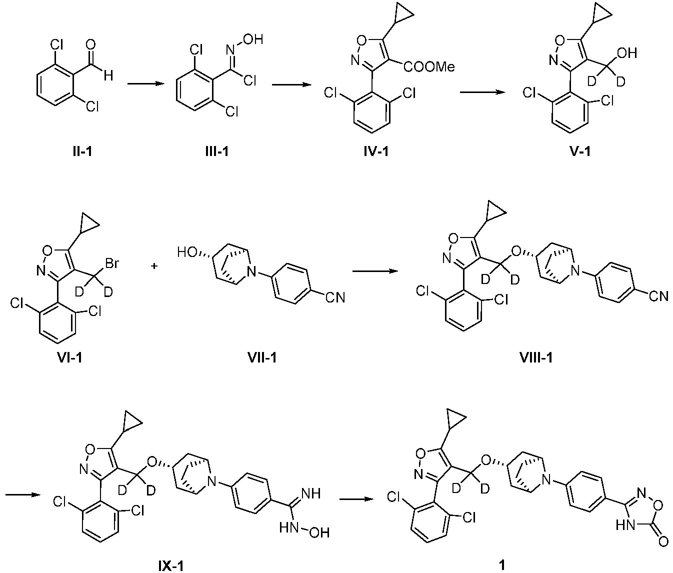

Aqueous potassium carbonate (3N, 182 mmol) was added dropwise to a solution of hydroxylamine hydrochloride (182 mmol) in ethanol (100 mL) with stirring at 0°C. 2,6-Dichlorobenzaldehyde II-1 (20 g, 114 mmol) was dissolved in 100 ml of ethanol, and then added to the above reaction solution. The temperature was raised to 90°C and the reaction was carried out for two hours. After the reaction was cooled to room temperature, it was concentrated to a solid. A solution of water/ethanol (1000 mL/100 mL) was added and stirred to break up the solid. The mixture was filtered and dried under vacuum at 50°C overnight to obtain the intermediate (18.4 g). The intermediate was dissolved in N,N-dimethylformamide (50 mL). A solution of N-chlorosuccinimide (97 mmol) in N,N-dimethylformamide (100 mL) was added dropwise at 0°C and stirred overnight. The reaction solution was poured into ice water at 0°C, then extracted with methyl tert-butyl ether (200 mL x 3 times). The organic phase was washed with saturated brine, and concentrated to obtain a crude product. n-Hexane (600 mL) was added to the flask containing the crude product, stirred with a magnetic bar, and filtered. The solid was dried under vacuum (30°C) to obtain intermediate **III-1** (18.3 g, yield 73%). ¹H NMR (400 MHz, CDCl₃) δ 7.43 - 7.39 (m, 2H), 7.39 - 7.33 (m, 1H).

Triethylamine (8.2 g) was added to methyl 3-cyclopropyl-3-oxopropanoate (82 mmol) and stirred for 30 minutes. The mixture was then cooled to 10°C, and a solution of **III-1** (18.3 g, 82 mmol) in anhydrous ethanol (80 mL) was added dropwise (internal temperature should not exceed 30°C), and the reaction was carried out at room temperature overnight. The reaction solution was diluted with Ethyl acetate (100 mL), and washed with water, and the aqueous phase was extracted with ethyl acetate (100 mL x 3 times). The organic phases were combined, washed with saturated brine, and concentrated. 100 mL of diethyl ether was added to the concentrate and stirred, and the solvent was removed under vacuum to obtain a solid product **IV-1** (21.6 g, yield 84%). ¹H NMR (400 MHz, CDCl₃) δ 7.43 - 7.39 (m, 2H), 7.39 - 7.33 (m, 1H), 3.72 (s, 3H), 2.21 - 2.09 (m, 1H), 1.35 - 1.28 (m, 2H), 1.25 - 1.18 (m, 2H); MS(ESI, m/z): 312[M+H]⁺.

Deuterated Lithium aluminum hydride (7.3g) was added to tetrahydrofuran (200mL) and cooled to 0°C. Then IV-1 (21.6 g, 69.2 mmol) in tetrahydrofuran (50 mL) was added dropwise (internal temperature should not exceed 5°C), and the reaction solution was stirred at room temperature for 2 h. The reaction was quenched by adding ice water (9 mL) at 0°C, then 15% aqueous sodium hydroxide solution (9 mL) and ice water (27 mL) were added dropwise and respectively. Afterwards, anhydrous magnesium sulfate (100 g) was added, and the above mixture was stirred at room temperature for 0.5 h, filtered and concentrated. The intermediate **V-1** (19 g, yield 96%) was obtained by column chromatography. ¹H NMR (400 MHz, DMSO-d₆) δ 7.72 - 7.59 (m, 2H), 7.58 - 7.50 (m, 1H), 4.90 (s, 1H), 2.38 - 2.26 (m, 1H), 1.16 - 1.03 (m, 4H). MS(ESI, m/z): 286[M+H]⁺.

**V-1** (19 g, 66.4 mmol) was dissolved in dichloromethane (200 mL), and cooled to 0°C. Phosphorus tribromide (66.4 mmol) was slowly added dropwise to the solution, and the reaction solution was stirred at room temperature for 2 h. The solvent was removed to obtain an oily substance, which was then diluted with ethyl acetate (100 mL), and the pH value of the reaction solution was adjusted to neutral with saturated aqueous sodium bicarbonate solution. The mixture was washed with water and the aqueous phase was extracted with ethyl acetate (100 mL x 3 times). The organic phases were combined, washed with saturated brine, and concentrated. Intermediate **VI-1** (20.2 g, yield 87%) was obtained by column chromatography. ¹H NMR (400 MHz, DMSO-d₆) δ 7.72 - 7.65 (m, 2H), 7.64 - 7.56 (m, 1H), 2.48 - 2.39 (m, 1H), 1.26 - 1.10 (m, 4H). MS(ESI, m/z): 348[M+H]⁺.

Potassium tert-butoxide (21.4 mmol) was added to a solution of **VII-1** (1.96 g, 8.6 mmol) in anhydrous tetrahydrofuran (150 mL) at 0°C, and stirred for 15 minutes. Then a solution of **VI-1** (8.6 mmol) in anhydrous tetrahydrofuran (50 mL) was added dropwise, and the reaction solution was stirred at room temperature for 4 h. Water (200 mL) was added to the reaction solution, and the mixture was extracted with ethyl acetate (200 mL x 3 times). The organic phase was washed with saturated brine, and concentrated. Intermediate **VIII-1** (2.1 g, 49% yield) was obtained by column chromatography. ¹H NMR (400 MHz, DMSO-*d*₆) δ 7.73-7.63 (m, 2H), 7.64 - 7.55 (m, 3H), 6.83 (*d*, *J* = 8.4 Hz, 2H), 4.16 (s, 2H), 3.37 (s, 1H), 2.38 - 2.31 (m, 1H), 1.83 - 1.74 (m, 6H), 1.56 - 1.49 (m, 2H), 1.16 - 1.06 (m, 4H). MS(ESI, m/z): 496[M+H]⁺.

VIII-1 (2.1 g, 4.2 mmol), hydroxylamine hydrochloride (8.4 mmol), absolute ethanol (80 mL) were added to a round bottom flask and stirred. Triethylamine (8.4 mmol) was slowly added dropwise. The mixture was heated to 80°C and reacted overnight. The mixture was cooled to room temperature. The solvent was removed and the residue was dissolved with dichloromethane (150 mL). The solution was washed with water and saturated brine, and the organic phase was concentrated. Intermediate **IX-1** (1.1 g, yield 50%) was obtained by silica gel column chromatography. ¹HNMR (400 MHz, DMSO-d₆) δ 7.73 - 7.63 (m, 2H), 7.64 - 7.55 (m, 3H), 6.83 (*d, J =* 8.4 Hz, 2H), 4.67 (s, 2H), 3.43 - 3.35 (m, 1H), 2.39 - 2.32 (m, 1H), 1.89 - 1.78 (m, 6H), 1.57 - 1.48 (m, 2H), 1.16 - 1.06 (m, 4H). MS(ESI, m/z): 529[M+H]⁺.

IX-1 (1.1 g, 2.1 mmol), N,N'-carbonyldiimidazole (3.1 mmol), 1,4-dioxane (100 mL) was added to a round bottom flask. Then 1,8-diazabicyclo[5.4.0]undec-7-ene (3.1 mmol) was added. The mixture was heated to 100°C and reacted for 8 hours. The reaction solution was cooled to room temperature, and diluted with water (100 mL), and the pH value adjusted to about 3 with 1M aqueous hydrochloric acid. Then the mixture was extracted with ethyl acetate (100 mL x 3 times). The organic phase were combined, washed with saturated brine, and concentrated to obtain a crude product. The final product 1 (158 mg, yield 13%) was obtained by silica gel column chromatography. ¹HNMR (400 MHz, DMSO-d₆) δ 7.65 - 7.63 (m, 2H), 7.59 - 7.55 (m, 3H), 6.83 (*d, J =* 8.4 Hz, 2H), 4.17 (s, 2H), 3.38 (s, 1H), 2.37 - 2.30 (m, 1H), 1.80 - 1.72 (m, 6H), 1.51 (*d, J =* 14.4 Hz, 2H), 1.16 - 1.06 (m, 4H). MS(ESI, m/z): 555[M+H]⁺.

### Example 2:

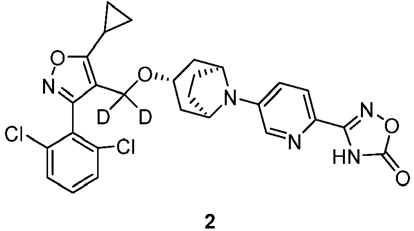

The synthesis of **example 2** was performed from intermediate **VII-2** through **route 1.** The synthetic route is as follows:

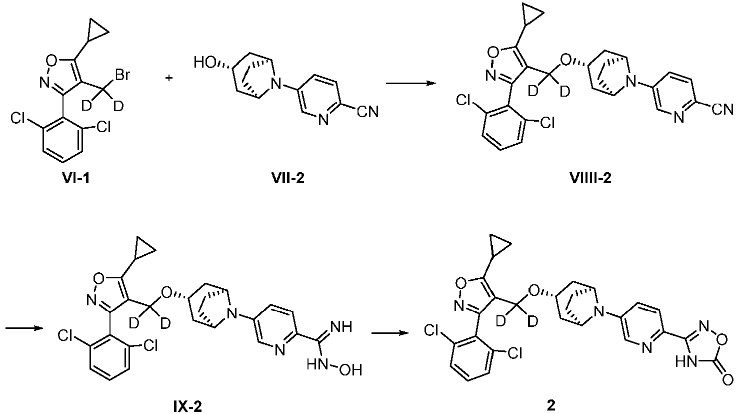

Starting from raw material **XIII-2,** compound **VII-2** was synthesized according to the synthetic method of intermediate **VII-1,** and then **2** was prepared through **route 1,** wherein:
The yield of white solid **VIII-2** was 77%. ¹HNMR (400 MHz, DMSO-*d*₆) δ8.21 (s, 1H), 7.70 - 7.62 (m, 3H), 7.60 - 7.54 (m, 1H), 7.16 (d, *J* = 8.4 Hz, 1H), 4.29 (s, 2H), 3.43 - 3.35 (m, 1H), 2.37 - 2.29 (m, 1H), 1.85-1.65 (m, 6H), 1.63 - 1.54 (m, 2H), 1.17 - 1.05 (m, 4H). MS(ESI, m/z): 497[M+H]⁺.
The yield of white solid **2** was 64%. ¹HNMR (400 MHz, DMSO-d₆) δ 8.20 - 8.16 (m, 1H), 7.70 (d, J = 8.4 Hz, 1H), 7.67 - 7.62 (m, 2H), 7.60 - 7.53 (m, 1H), 7.28 - 7.21 (m, 1H), 4.28 (s,br, 2H), 3.42 - 3.40 (m, 1H), 2.38 - 2.29 (m, 1H), 1.81 - 1.69 (m, 6H), 1.60 - 1.52 (m, 2H), 1.16 - 1.05 (m, 2H). MS(ESI, m/z): 556[M+H]⁺.

### Example 3

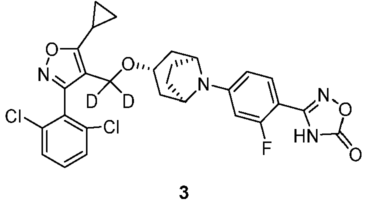

The synthesis of **Example 3** was performed from intermediate **VII-3** through **route 1,** and the synthetic route is as follows:

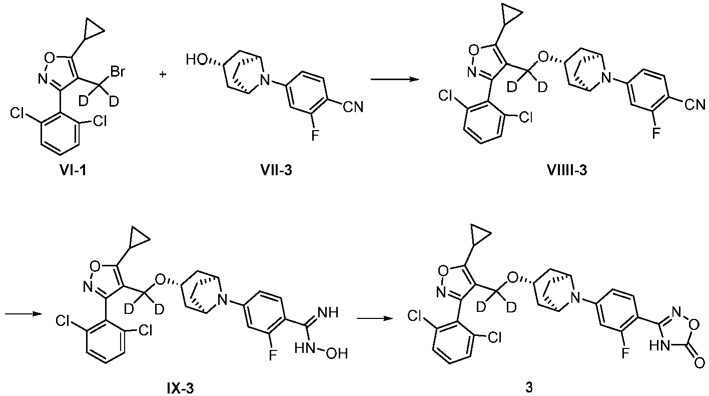

Starting from raw material **XIII-3,** compound **VII-3** was synthesized according to the synthetic method of intermediate **VII-1,** and then **3** was prepared through **route 1,** wherein:
The yield of white solid **VIII-3** was 67%. HNMR (400 MHz, DMSO-d₆) δ7.68 - 7.45 (m, 4H), 6.69 (t, *J* = 12.0 Hz, 2H), 4.18 (s, 2H), 3.46 - 3.36 (m, 1H), 2.38 - 2.27 (m, 1H), 1.80 - 1.66 (m, 6H), 1.60 - 1.51 (m, 2H), 1.20 - 1.03 (m, 4H). MS(ESI, m/z): 497[M+H]⁺.
The yield of white solid 3 is 38%.¹H NMR (400 MHz, DMSO-*d*₆) δ7.69 - 7.42 (m, 4H), 6.74 (d, *J* = 13.6 Hz, 1H), 6.63 (d, *J* = 8.8 Hz, 1H), 4.21 (s, 2H), 3.47 - 3.43 (m, 1H), 2.35 - 2.30 (m, 1H), 1.76 - 1.70 (m, 6H), 1.58 - 1.50 (m, 2H), 1.20 - 1.06 (m, 4H). MS(ESI, m/z): 573[M+H]⁺.

### Example 4:

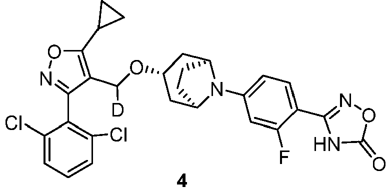

Synthesis of **Example 4** was performed with reference to the operation of **Example 3,** and **4** was prepared from intermediate **IV-1** through **route 2.** The synthetic route is as follows:

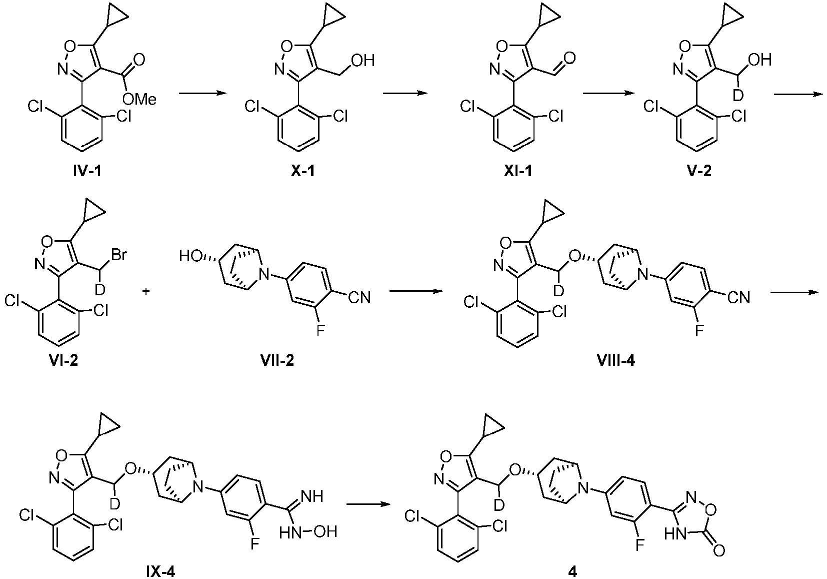

Lithium aluminum hydride (420 mg, 10 mmol) was added to tetrahydrofuran (8 mL) and cooled to 0°C. Then **IV-1** (2 mmol) in tetrahydrofuran (2 mL) was added dropwise (internal temperature shall not exceed 5°C), and the reaction solution was stirred at room temperature for 2 h. The reaction was quenched by adding ice water (0.4 mL) at 0°C, then 15% aqueous sodium hydroxide solution (0.4 mL) and ice water (1.2 mL) were added dropwise. Then anhydrous magnesium sulfate (8 g) was added. The mixture was stirred at room temperature for 0.5 h, filtered and concentrated. Intermediate **X-1** (1 g, yield 84%) was obtained by column chromatography. MS(ESI, m/z): 284[M+H]⁺.

**X-1** (1 g, 3.53 mmol) was added to dichloromethane (20 mL), and pyridinium chlorochromate (14.14 mmol) was added at room temperature. The reaction solution was stirred at room temperature for 1 h, then filtered and concentrated. Intermediate XI-1 (870 mg, yield 88%) was obtained by column chromatography. MS(ESI, m/z): 282[M+H]⁺.

Lithium aluminum hydride (260 mg, 6.2 mmol) was added to tetrahydrofuran (4 mL) and cooled to 0°C. Then **XI-1** (3.1 mmol) in tetrahydrofuran (1 mL) was added dropwise (internal temperature shall not exceed 5°C), and the reaction solution was stirred at room temperature for 2 h. The reaction was quenched by adding ice water (0.2 mL) at 0°C, then 15% aqueous sodium hydroxide solution (0.2 mL) and ice water (0.6 mL) were added dropwise, respectively. Then anhydrous magnesium sulfate (10 g) was added, and the above mixture was stirred at room temperature for 0.5 h, filtered and concentrated. Intermediate **V-2** (730 mg, yield 83%) was obtained by column chromatography. ¹H NMR (400 MHz, DMSO-d₆) δ 7.62-7.60 (m, 2H), 7.56-7.52 (m, 1H), 4.92 (d, *J* = 5.2 Hz, 1H), 4.18 (d, *J* = 5.2 Hz, 1H), 2.35 - 2.28 (m, 1H), 1.14 - 1.04 (m, 4H). MS(ESI, m/z): 285[M+H]⁺.

**V-2** (730 mg, 2.57 mmol) was dissolved in dichloromethane (10 mL) and cooled to 0°C. Phosphorus tribromide (3.08 mmol) was slowly added dropwise to the solution, and the reaction solution was stirred at room temperature for 2 h. The solvent was removed from the reaction solution to obtain an oily substance, which was diluted with ethyl acetate (20 mL). The pH of the reaction solution was adjusted to neutral with saturated aqueous sodium bicarbonate solution. The mixture was washed with water, and the aqueous phase was extracted with ethyl acetate (100 mL x 3 times). The organic phases were combined, washed with saturated brine, and concentrated. Intermediate **VI-2** (720 mg, 81% yield) was obtained by column chromatography. MS(ESI, m/z): 347[M+H]⁺.

Potassium tert-butoxide (4.16 mmol) was added to a solution of **VII-2** (512 mg, 2.08 mmol) in anhydrous tetrahydrofuran (10 mL) at 0°C, and the solution was stirred for 15 minutes. Then a solution of VI-2 (2.08 mmol) in anhydrous tetrahydrofuran (5 mL) was added dropwise, and the reaction solution was stirred at room temperature for 4 h. Water (20 mL) was added to the reaction solution. The solution was extracted with ethyl acetate (20 mL x 3 times). The organic phase was washed with saturated brine and concentrated. Intermediate **VIII-4** was obtained by column chromatography (420 mg, yield 39%). MS(ESI, m/z): 513[M+H]⁺.

**VIII-4** (420 mg, 0.82 mmol), hydroxylamine hydrochloride (1.64 mmol), absolute ethanol (5 mL) were added to a round bottom flask and stirred. Triethylamine (1.64 mmol) was slowly added dropwise. The mixture was heated to 80°C and reacted overnight, then cooled to room temperature. The solvent was removed, and the residue was dissolved with dichloromethane (20 mL). The solution was washed with water and saturated brine, and then the organic phase was concentrated. Intermediate **IX-4** (360 mg, yield 81%) was obtained by silica gel column chromatography. MS(ESI, m/z): 546[M+H]⁺.

**IX-4** (360 mg, 0.66 mmol), N,N'-carbonyldiimidazole (0.99 mmol), 1,4-dioxane (5 mL) was added to a round bottom flask, then 1,8-diazabicyclo[5.4.0]undec-7-ene (0.99 mmol) was added. The solution was heated to 100°C and reacted for 8 hours, then cooled to room temperature. The mixture was diluted with water (10 mL), and the pH value was adjusted to about 3 with 1M aqueous hydrochloric acid. The mixture was then extracted with ethyl acetate (10 mL x 3 times). The organic phases were combined, washed with saturated brine, and concentrated. The final product 4 (76.7 mg, 20% yield) was obtained by silica gel column chromatography. ¹HNMR (400 MHz, DMSO-d₆) δ7.65-7.62 (m, 2H), 7.58 - 7.55 (m, 1H), 7.47 (t, *J =* 8.6 Hz, 1H), 6.72 - 6.65 (m, 2H), 4.22 (s, 1H), 4.17 (s, 2H), 3.39 (s, 1H), 2.36 - 2.30 (m, 1H), 1.76-1.73 (m, 6H), 1.52 (d, *J* = 14.8 Hz, 2H), 1.14 - 1.08 (m, 4H). MS(ESI, m/z): 572[M+H]⁺.

### Example 5:

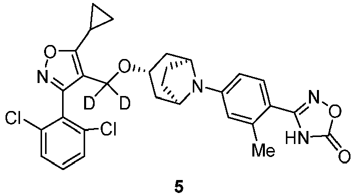

The synthesis of **Example 5** was performed from intermediate **VII-4** through route 1, and the synthetic route is as follows:

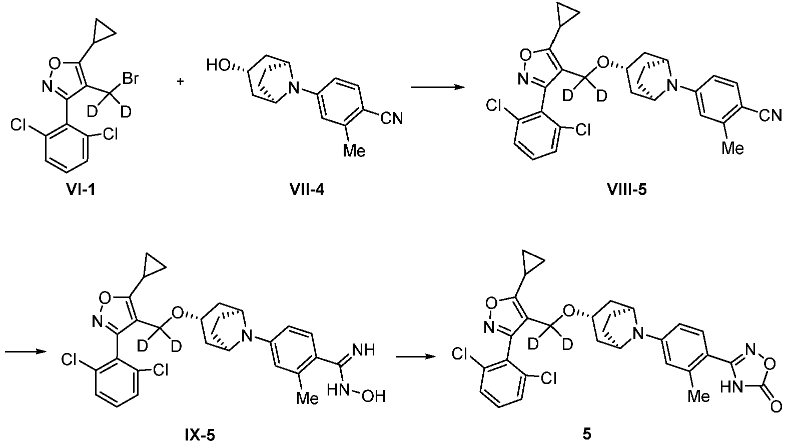

Starting from raw material **XIII-4,** compound **VII-4** was synthesized through the synthetic method of synthesizing intermediate **VII-1,** and then **5** was prepared through **route 1,** wherein:
The yield of white solid **VIII-5** was 47%. ¹HNMR (400 MHz, DMSO-d₆) δ7.68 - 7.61 (m, 2H), 7.60 - 7.55 (m, 1H), 7.42 (d, *J =* 8.4 Hz, 1H), 6.71 (s, br, 1H), 6.62 (d, *J* = 8.8 Hz, 1H), 4.17 (s, 2H), 3.41 - 3.36 (m, 1H), 2.39 - 2.26 (m, 4H), 1.84 - 1.66 (m, 6H), 1.58 - 1.46 (m, 2H), 1.19 - 1.02 (m, 4H). MS(ESI, m/z): 510[M+H]⁺.
The yield of white solid **5** was 11%. ¹H NMR (400 MHz, DMSO-d₆) δ7.69 - 7.62 (m, 2H), 7.60 - 7.35 (m, 2H), 6.75 - 6.545 (m, 2H), 4.19 (s, 2H), 3.43 - 3.33 (m, 1H), 2.41 - 2.29 (m, 4H), 1.88 - 1.66 (m, 6H), 1.60 - 1.46 (m, 2H), 1.21 - 1.03 (m, 4H). MS(ESI, m/z): 569[M+H]⁺.

### Example 6:

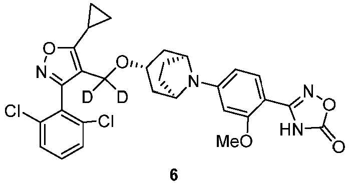

The synthesis of **Example 6** was performed from intermediate **VII-5** through **route 1,** and the synthetic route is as follows:

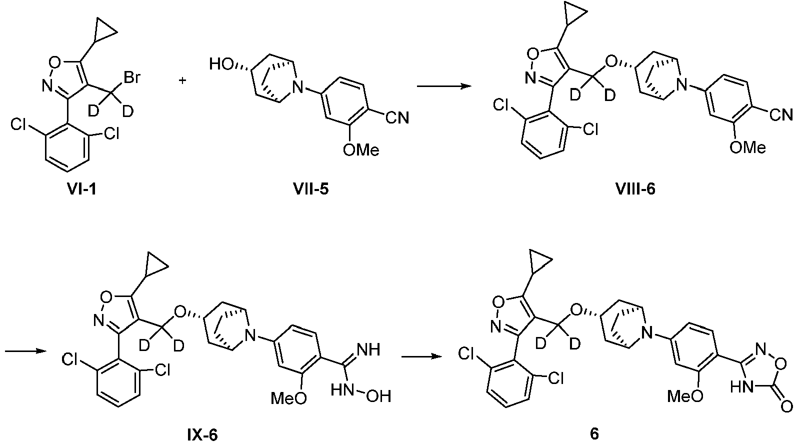

Starting from raw material **XIII-5,** compound **VII-5** was synthesized through the synthetic method of synthesizing intermediate **VII-1,** and then 6 was prepared through route 1, wherein:
The yield of white solid **VIII-6** was 32%.¹HNMR (400 MHz, DMSO-*d*₆) δ7.66 - 7.61 (m, 2H), 7.60 - 7.52 (m, 1H), 7.32 (d, *J =* 8.4 Hz, 1H), 6.39 - 6.29 (m, 2H), 4.22 (s, 2H), 3.83 (s, 3H), 3.41 - 3.32 (m, 1H), 2.36 - 2.28 (m, 1H), 1.84 - 1.66 (m, 6H), 1.60 - 1.49 (m, 2H), 1.20 - 1.02 (m, 4H). MS(ESI, m/z): 526[M+H]⁺.
The yield of white solid 6 was 9%. ¹H NMR (400 MHz, DMSO-d₆) δ7.69 - 7.52 (m, 3H), 7.37 - 7.29 (m, 1H), 6.45 - 6.36 (m, 2H), 4.28 (s, 2H), 3.87 (s, 3H), 3.43 - 3.32 (m, 1H), 2.42 - 2.27 (m, 1H), 1.87 - 1.64 (m, 6H), 1.61 - 1.47 (m, 2H), 1.21 - 1.02 (m, 4H). MS(ESI, m/z): 585[M+H]⁺.

### Example 7:

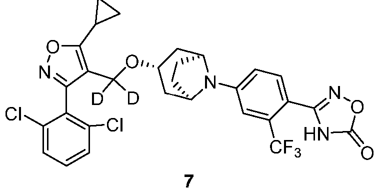

The synthesis of **Example 7** was performed from intermediate **VII-6** through **route 1,** and the synthetic route is as follows:

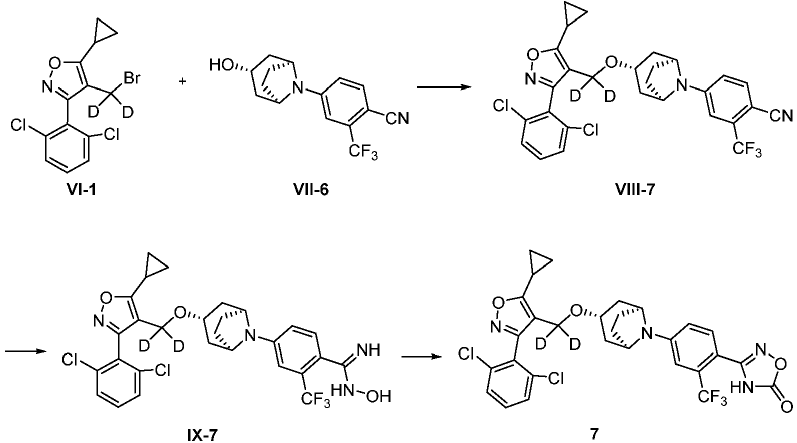

Starting from raw material XIII-6, compound **VII-6** was synthesized through the synthetic method of synthesizing intermediate **VII-1,** and then 7 was prepared through route **1,** wherein:
The yield of white solid **VIII-7** was 56%.¹HNMR (400 MHz, DMSO-d₆) δ7.75 (d, *J* = 9.2 Hz, 1H), 7.67 - 7.52 (m, 3H), 7.08 (s, 1H), 7.02 (d, *J =* 8.8 Hz, 1H), 4.32 (s, 2H), 3.43 - 3.32 (m, 1H), 2.36 - 2.28 (m, 1H), 1.78 - 1.69 (m, 3H), 1.61 - 1.50 (m, 2H), 1.16 - 1.06 (m, 4H). MS(ESI, m/z): 564[M+H]⁺.
The yield of white solid 7 was 49%. ¹H NMR (400 MHz, DMSO-d₆) δ 7.67 - 7.47 (m, 4H), 7.11 - 7.04 (m, 2H), 4.27 (s, 2H), 3.48 - 3.44 (m, 1H), 2.35 - 2.31 (m, 1H), 1.85 - 1.69 (m, 6H), 1.62 - 1.48 (m, 2H), 1.20 - 1.01 (m, 4H). MS(ESI, m/z): 623[M+H]⁺.

### Example 8:

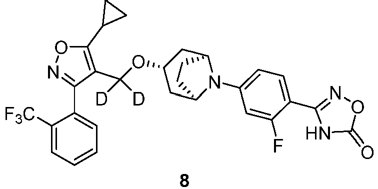

The synthesis of **Example 8** was performed from intermediate **II-2** through **route 1,** and the synthetic route is as follows:

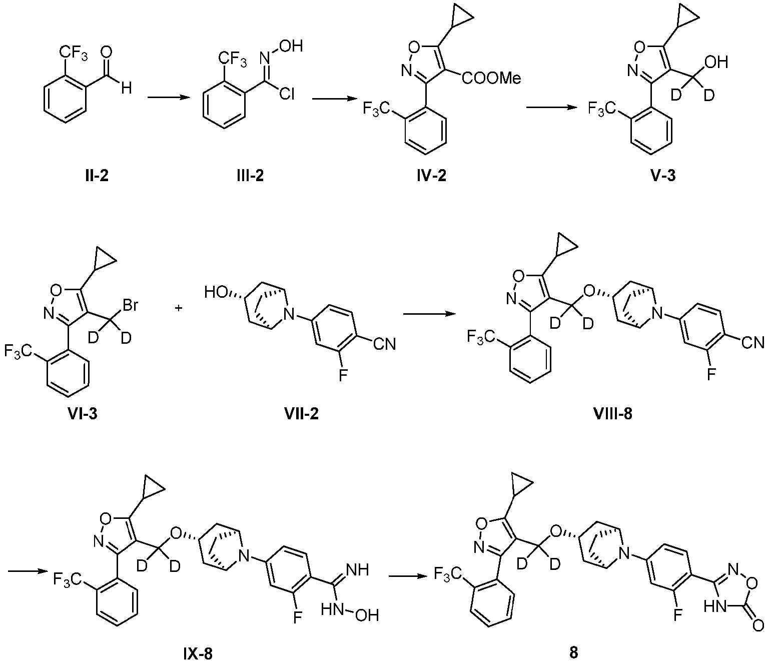

Wherein:
The yield of colloid **V-3** was 98%.¹HNMR (400 MHz, DMSO-d₆) δ7.88 *(d, J =* 7.6 Hz, 1H), 7.81 - 7.71 (m, 2H), 7.59 *(d, J =* 7.2 Hz, 1H), 4.90 (s, 1H), 2.33-2.26 (m, 1H), 1.12-1.05 (m, 4H). MS(ESI, m/z): 286[M+H]⁺.
The yield of white solid **VIII-8** was 31%.¹HNMR (400 MHz, DMSO-d₆) δ7.90 (d, *J* = 7.6 Hz, 1H), 7.81-7.72 (m, 2H), 7.58 (d, *J* = 7.6 Hz, 1H), 7.50 (t, *J* = 8.4 Hz, 1H), 6.74 (dd, *J =* 13.8, 2.2 Hz, 1H), 6.63 (dd, *J =* 8.6, 2.2 Hz, 1H), 4.20 (s, 1H), 3.61 - 3.57 (m, 1H), 3.40 - 3.32 (m, 1H), 2.33 - 2.29 (m, 1H), 1.76-1.69 (m, 6H), 1.54 (d, *J* = 14.4 Hz, 2H), 1.18 - 1.05 (m, 4H). MS(ESI, m/z): 514[M+H]⁺.
The yield of white solid **8** was 29%. ¹HNMR (400 MHz, DMSO-d₆) δ7.90 (d, *J =* 8.0 Hz, 1H), 7.81 - 7.72 (m, 2H), 7.58 (d, *J =* 7.2 Hz, 1H), 7.47 (t, *J =* 8.6 Hz, 1H), 6.72 - 6.66 (m, 2H), 4.17 (s, 2H), 3.38 (s, 1H), 2.34 - 2.28 (m, 1H), 1.77 - 1.73 (m, 6H), 1.52 (d, *J* = 14.8 Hz, 2H), 1.13 - 1.05 (m, 4H). MS(ESI, m/z): 573[M+H]⁺.

### Example 9:

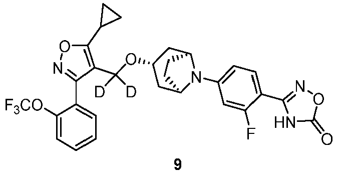

The synthesis of **Example 9** was performed from intermediate **II-3** through **route 1,** and the synthetic route is as follows:

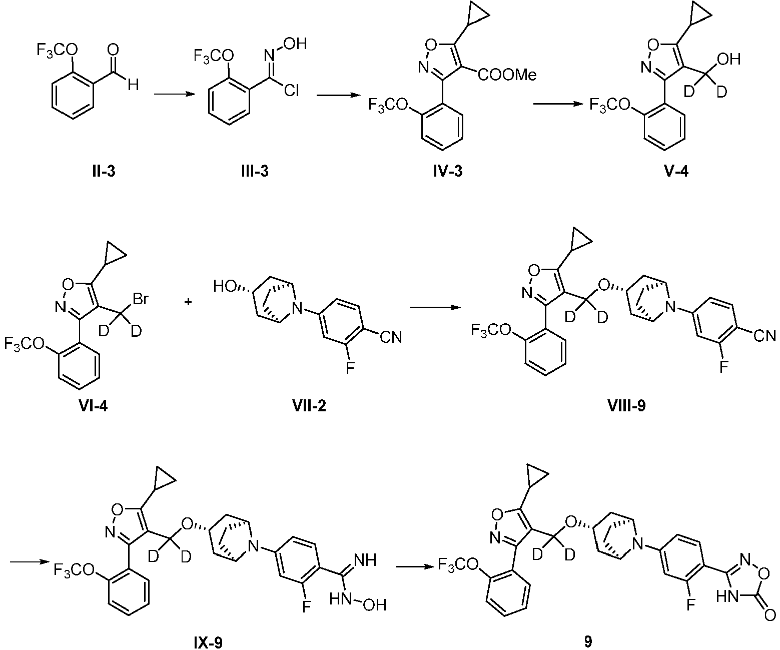

Wherein:
The yield of colloid **V-4** was 76%.¹HNMR (400 MHz, DMSO-d₆) δ 7.67-7.63 (m, 2H), 7.53 (d, *J* = 6.8 Hz, 2H), 4.96 (s, 1H), 2.32-2.27 (m, 1H), 1.13-1.04 (m, 4H). MS(ESI, m/z): 302[M+H]⁺.
The yield of colloid **VIII-9** was 47%.¹HNMR (400 MHz, DMSO-d₆) δ 7.69 - 7.61 (m, 2H), 7.56 - 7.49 (m, 3H), 6.75 *(d, J=* 14.0 Hz, 1H), 6.64 (*d, J* = 8.8 Hz, 1H), 4.21 (s, 2H), 3.45 - 3.43 (m, 1H), 2.36 - 2.29 (m, 1H), 1.77 - 1.69 (m, 6H), 1.56 (*d, J* = 14.8 Hz, 2H), 1.23 - 1.06 (m, 4H). MS(ESI, m/z): 530[M+H]⁺.
The yield of white solid 9 was 21%.¹HNMR (400 MHz, DMSO-d₆) δ7.69 - 7.62 (m, 2H), 7.56 - 7.46 (m, 3H), 6.72 - 6.66 (m, 2H), 4.18 (s, 2H), 3.43 (s, 1H), 2.35 - 2.31 (m, 1H), 1.80 - 1.75 (m, 6H), 1.54 *(d, J* = 14.4 Hz, 2H), 1.13 - 1.06 (m, 4H). MS(ESI, m/z): 589[M+H]⁺.

### Pharmacological Experimental Example

### Methods for Detecting FXR Agonistic Activity of Compounds Based on Reporter Gene Activity Detection Methods:

### 1.1 Construction and Preparation of Plasmids pGAL4-FXR-LBD and pG5-Luc

The pGAL4-FXR-LBD and pG5-Luc plasmids used in the reporter gene detection system were constructed according to conventional molecular cloning methods, including main steps:
the FXR (NM_001206979.2) cDNA sequence corresponding to the amino acid sequence of FXR-LBD (212-476AA) was inserted into pGAL4 vector between BamHI and NotI restriction sites using PCR technology to obtain pGAL4-FXR-LBD;
pG5-Luc and phRL-TK plasmids were donated by Shanghai Institute of Materia Medica, Chinese Academy of Sciences;
the plasmid was transformed into DH5α *Escherichia coli* by the CaCl₂ method, and after further culture and amplification, the corresponding plasmid DNA was purified and obtained with a plasmid extraction kit (TIANGEN, #D107).

### 1.2 Plasmid Co-transfection of HEK293T Cells and Compound Treatment

HEK293T cells were seeded in 96-well plates at a density of 1×10⁴/well one day before plasmid transfection. Cell transfection was performed according to the instructions of the transfection reagent FuGENE^{®} HD (Promega, #E2311), including main steps:
taking 1 well as an example, plasmids pGAL4-FXR-LBD, pG5-Luc and phRL-TK were added to 10 uL of Opti-MEM^{™} I medium (Gibco, #11058021) at 20 ng, 50 ng and 5 ng and mixed well;
0.25 uL of FuGENE^{®} HD was added, mixed well and let stand for 5 min at room temperature;
then 10 uL of this mixture was added to the cell well containing 100 uL of culture medium.

6 h after cells were co-transfected, the compounds were diluted to 10 concentrations with a 3-fold gradient, with 1 uM as the highest concentration. Then the diluted compounds were added to the cell culture medium for treatment for 24 h. 2 duplicate wells were set up in total. Compound LJN452 was used as positive control.

### 1.3 Dual-Glo Luciferase Detection

After cells were treated with compounds for 24 h, detections were performed according to the instructions of the Dual-Glo^{®} Luciferase Assay System (Promega, #E2940), including main steps:
50 uL of culture medium were aspirated and discarded per well, and 50 uL of Dual-Glo^{®} Luciferase reagent were added, then shaken at room temperature for 10 min;
80 uL of the lysis reaction solution were taken to a white opaque optiPlate-96 well plate, and the luminescence signal value of Firefly luciferase (Firefly-Luc) was detected by a MD i3x multifunctional microplate reader;
then 40 uL of Dual-Glo^{®} Stop & Glo^{®} reagent were added and shaken at room temperature for 10 min;
the luminescence signal value of Renilla luciferase (Renilla-Luc) was detected by the MD i3x multifunctional microplate reader;
The ratio of Firefly-Luc/Renilla-Luc was used as the activating activity of the compound on FXR, and normalized by the ratio of the solvent DMSO group. The dose-response curves were fitted with four parameters using GraphPad Prism 6.0 software, and EC50 values were calculated.

### 2.Results

The experimental data show that the compounds all have certain FXR agonistic activity. Wherein the EC₅₀ values of **Examples 1, 2, 3,** and **4** are all less than 5 nM, which have very strong FXR agonistic activity. The FXR agonistic activity data of other examples are shown in **Table 1.**

**Table 1 FXR agonistic activity of compounds**

| **Samples** | **Cellular Level Activity EC50(nM) of FXR** |
|---|---|
| Example 1 | **** |
| Example 2 | **** |
| Example 3 | **** |
| Example 4 | **** |
| Example 5 | *** |
| Example 6 | *** |
| Example 7 | *** |
| Example 8 | *** |
| Example 9 | *** |
| LJN452 | *** |
| Control 1 | *** |

| | |
|---|---|
| ****: EC₅₀(nM) < 5; ***: 5 < EC₅₀(nM) < 10; **: 10 < EC₅₀(nM) < 50; *: 50 < EC₅₀(nM). | |

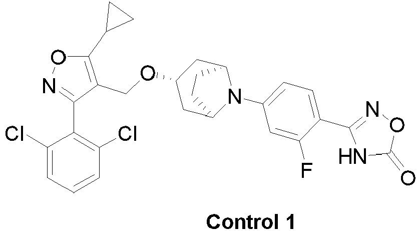

The results show that the compounds of the present invention exhibited better cellular level activity than the existing FXR agonist compound LJN452 and the non-deuterated **Control 1.** Especially, the compound of **Example 3** of the present application is the deuterated compound of **Control 1,** which shows significantly improved activity, suggesting that this position is a key deuterated site for this type of compound.

### Pharmacokinetic evaluation:

Mice were used to compare the bioavailability and pharmacokinetic behaviors of deuterated embodiment 1 and non-deuterated embodiment 2. In each group of embodiment, 6 male ICR mice with a similar body weight were selected, of which 3 mice were dosed orally in a single dose of 3 mg/kg, and 3 mice were dosed intravenously in a single dose of 1 mg/kg. Blood samples were collected at time points of 15 min, 30 min, 1 h, 2 h, 4 h and 7 h after administration. The concentrations of plasma samples were analyzed by LC-MS/MS. A free tool of PKSolver and the non-compartment model (NCA) ware applied to analyze the pharmacokinetic parameters of compounds, as shown in Table 1 below.

### Experiment scheme:

### Experiment compounds: embodiment 1 and embodiment 2.

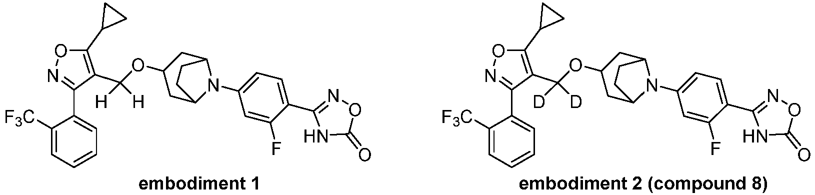

### Laboratory animal:

12 healthy male ICR mice, commercially available from Charles River with an animal production license NO.: 2021123 1Abzz0619000376, were divided into 4 groups, 3 in each group.

Formulation preparation: a certain amount of compounds were weighed and added into a 2% DMSO+15% Solutol+83% saline, to prepare a clear solution.

**Dosage:** ICR mice were fasted overnight and given each compound at an oral dose of 3 mg/kg or an intravenous dose of 1 mg/kg. The administrated volume for oral and intravenous administration are 10 mL/kg and 5 mL/kg, respectively. Food was withheld until 2 h post-dose.

**Sample collection:** About 30 µL of blood was collected via great saphenous vein at 15 min, 30 min, 1 h, 2 h, 4 h and 7 h after dosage. The blood was placed into a commercial tube containing K₂-EDTA. Plasma samples were then obtained by centrifuging the blood samples at approximately 4°C, 4600 rpm for 5 minutes. All plasma samples were then quickly frozen on dry ice and kept at -70°C until LC-MS/MS analysis.

**Sample preparation:** 10 µL of plasma samples were precipitated by methanol containing 50 nmol/L of α-naphthoflavone as internal standard. The mixture was votex-mixed well and centrifuged at 14000 rpm for 5 min at 4°C. 75 µL of the supernatant was taken and mixed with 75 µL of methanol, and introduced for LC-MS/MS analysis.

Results of the pharmacokinetic parameters are shown in Table 1.

**Table 1**

| **Pharmacokinetic Parameters in Mice** | | | | | | | | |
|---|---|---|---|---|---|---|---|---|
| Compound | Specie s | F(% ) | Intravenous administration (1 mg/kg) | | | Oral administration (3 mg/kg) | | |
| | | | T_{1/2}(h) | CL_{_obs(mL/mi n/kg)} | Vss_{_obs}(mL/ kg) | Tₘₐₓ(h ) | Cₘₐₓ(ng/m L) | AUC_{0-7 h}(h*ng/mL) |
| Embodime nt 1 | Mice | 12.6 | 0.26±0. 01 | 20.5±4.6 | 418±76 | 0.5±0. 0 | 312±140 | 318±172 |
| Embodime nt 2 | Mice | 25.2 | 0.47±0. 08 | 21.6±2.4 | 704±33 | 0.5±0. 0 | 555±25 | 587±54 |

| | | | | | | | | |
|---|---|---|---|---|---|---|---|---|
| **Conclusion:** the compound from embodiment 2 possess better absorption and higher bioavailability than the compound from embodiment 1, and selected for further investigation. | | | | | | | | |

It should be understood that after reading the above taught content of the present invention, those skilled in the art can make various changes or modifications to the present invention, and these equivalent forms also fall within the scope defined by the appended claims of the present application.

## Claims

1. A compound of formula I: 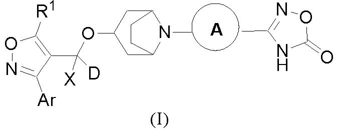 or an enantiomer, diastereomer, tautomer, racemate, hydrate, solvate, or pharmaceutically acceptable salt thereof;
wherein:
Ar is selected from the group consisting of a substituted or unsubstituted C₆-C₁₀ aryl, and substituted or unsubstituted 5-9 membered heteroaryl ring (including a single ring or fused ring, and containing 1-3 heteroatoms selected from O, S or N);
A is selected from the group consisting of a substituted or unsubstituted C₆-C₁₀ aryl, substituted or unsubstituted 5-9 membered heteroaryl ring (including a single ring or fused ring, and containing 1-3 heteroatoms selected from O, S or N);
R¹ is selected from the group consisting of a substituted or unsubstituted C₁-C₆ alkyl, substituted or unsubstituted C₃-C₆ cycloalkyl, substituted or unsubstituted 5-9 membered heterocyclic group (containing 1-3 heteroatoms selected from O, S or N);
X is selected from the group consisting of H and D;
wherein, the "substituted" means that one or more hydrogen atoms on a group are each independently replaced by a substituent selected from the group consisting of a halogen, halogenated C₁-C₆ alkyl, halogenated C₁-C₆ alkoxy, C₁-C₆ alkyl, C₁-C₆ alkoxy, C₃-C₆ cycloalkyl, C₃-C₆ cycloalkoxy, cyano and nitro.

2. The compound of claim **1,** wherein R¹ is selected from the group consisting of a substituted or unsubstituted C₁-C₆ alkyl, and substituted or unsubstituted C₃-C₆ cycloalkyl;
wherein the "substituted" means that one or more hydrogen atoms on a group are each independently replaced by a substituent selected from the group consisting of a halogen, halogenated C₁-C₆ alkyl, halogenated C₁-C₆ alkoxy, C₁-C₆ alkyl, C₁-C₆ alkoxy, C₃-C₆ cycloalkyl, C₃-C₆ cycloalkoxy, cyano and nitro.

3. The compound of claim **1,** wherein Ar is selected from the group consisting of a substituted or unsubstituted C₆-C₁₀ aryl, substituted or unsubstituted 5-9 membered heteroaryl ring;
and, the substituent is selected from the group consisting of H, F, Cl, Br, methyl, ethyl, n-propyl, isopropyl, n-butyl, isobutyl, trifluoromethyl, and trifluoromethoxy.

4. The compound of claim **1,** wherein A is selected from the group consisting of a substituted or unsubstituted C₆-C₁₀ aryl, substituted or unsubstituted 5-9 membered heteroaryl;
and, the substituent on the aryl or heteroaryl is selected from the group consisting of H, F, Cl, Br, methyl, ethyl, n-propyl, isopropyl, n-butyl, isobutyl, trifluoromethyl, and trifluoromethoxy.

5. The compound of claim **1,** wherein R¹ is selected from the group consisting of a methyl, ethyl, n-propyl, isopropyl, n-butyl, isobutyl, cyclopropyl, cyclobutyl and cyclopentyl.

6. The compound of claim **1,** which is selected from the group consisting of

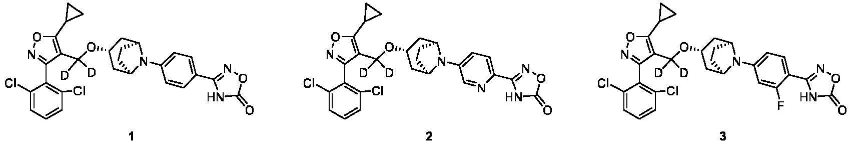

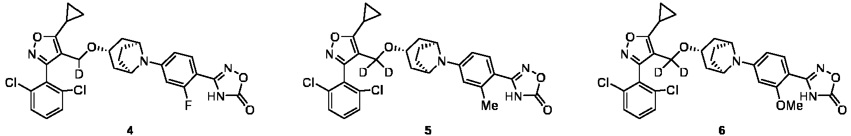

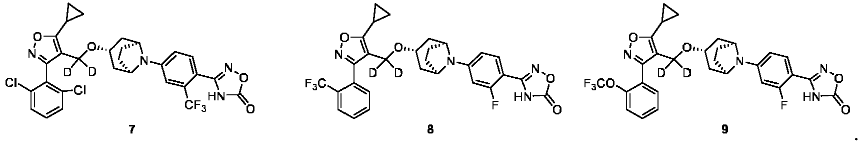

7. A preparation method of the compound of claim 1, comprising preparing a compound of formula I by a method selected from the following route 1 or 2: 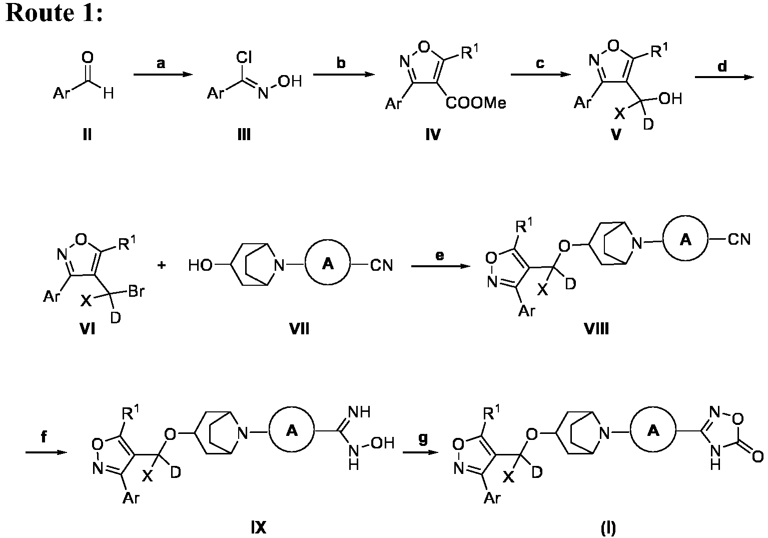
(a) a substituted benzaldehyde of formula **II,** as starting material, is reeacted with hydroxylamine hydrochloride under the presence of a base to obtain the intermediate; the intermediate is then chlorinated by N-chlorosuccinimide (NCS) to form a compound of general formula **III;**
(b) the compound of formula **III** is then reacted with the corresponding 3-oxopropionate under the presence of a base to obtain a compound of formula **IV;**
(c) the ester compound of formula **IV** is reduced to the corresponding alcohol, i.e. a compound of formula **V,** under the presence of a deuterated reducing agent;
(d) the compound of formula **V** is brominated by bromine reagent to obtain a compound of **VI;**
(e) the compound of formula **VI** and the compound of formula **VII** are reacted under the presence of a base to obtain a compound of formula **VIII;**
(f) the compound of formula **VIII** is reacted with hydroxylamine hydrochloride under the presence of a base to obtain a compound of formula **IX;**
(g) the compound of formula **IX** is reacted under the presence of a phosgene, triphosgene or carbonyldiimidazole to obtain the compound of formula **I;**
wherein X is a deuterium; and
R¹, Ar, A are determined as described in claim **1;** 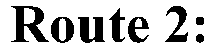 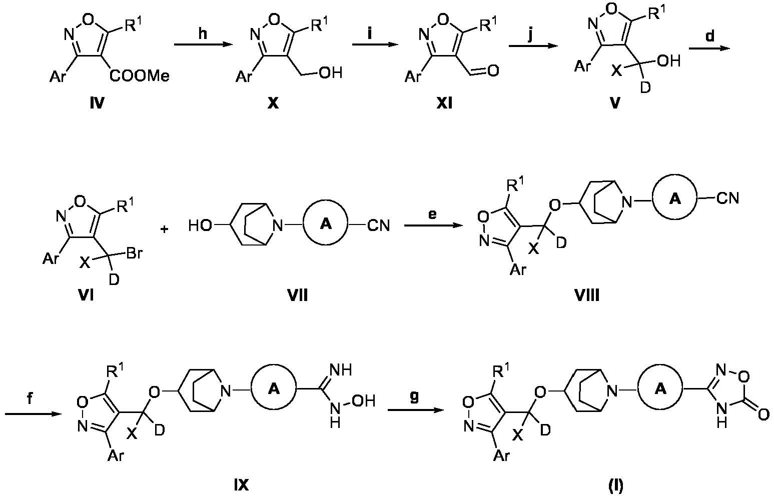
(a) an ester compound of formula **IV** is reduced to the corresponding alcohol, i.e. a compound of formula **X,** under the presence of a reducing agent;
(b) the compound of formula **X** is oxidized to the corresponding aldehyde, i.e. a compound of formula **XI,** under the action of an oxidizing agent;
(c) the ester compound of formula **XI** is reduced to a compound of formula V under the presence of a deuterated reducing agent;
(d) the compound of formula **V** is brominated by bromine reagent to obtain a compound of **VI;**
(e) the compound of formula **VI** and a compound of formula **VII** are reacted under the presence of a base to obtain a compound of formula **VIII;**
(f) the compound of formula **VIII** is reacted with hydroxylamine hydrochloride under the presence of a base to obtain a compound of formula **IX;**
(g) the compound of formula **IX** is reacted under the presence of a phosgene, triphosgene or carbonyldiimidazole to obtain the compound of formula **I;**
wherein X is a hydrogen; and
R¹, Ar, A are determined as described in claim **1.**

8. A pharmaceutical composition comprising the compound of formula **I** of claim 1, or an enantiomer, diastereomer, tautomer, racemate, hydrate, solvate, or pharmaceutically acceptable salt thereof;
and a pharmaceutically acceptable carrier.

9. Use of the compound of formula **I** of claim 1, or an enantiomer, diastereomer, tautomer, racemate, hydrate, solvate, or pharmaceutically acceptable salt thereof for the preparation of a pharmaceutical composition for treating diseases or conditions related to FXR activity or expression.

10. The use of claim **9,** wherein the diseases related to FXR is selected from the group consisting of diseases related to bile acid metabolism, glucose metabolism, lipid metabolism, inflammation, and/or liver fibrosis process.

## Patentansprüche

1. Verbindung der Formel I: 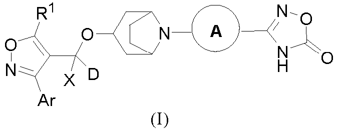
oder Enantiomer, Diastereomer, Tautomer, Racemat, Hydrat, Solvat oder pharmazeutisch annehmbares Salz davon;
wobei:
Ar aus der Gruppe ausgewählt ist, die aus einem substituierten oder unsubstituierten C₆-C₁₀-Aryl und einem substituierten oder unsubstituierten fünf- bis neungliedrigen Heteroarylring besteht (einschließlich eines einzelnen Rings oder kondensierten Rings, der 1-3-Heteroatome enthält, die aus O, S oder N ausgewählt sind);
A aus der Gruppe ausgewählt ist, die aus einem substituierten oder unsubstituierten C₆-C₁₀-Aryl, einem substituierten oder unsubstituierten fünf- bis neungliedrigen Heteroarylring besteht (einschließlich eines einzelnen Rings oder kondensierten Rings, der 1-3 Heteroatome enthält, die aus O, S oder N ausgewählt sind);
R¹ aus der Gruppe ausgewählt ist, die aus einer substituierten oder unsubstituierten C₁-C₆-Alkyl-, substituierten oder unsubstituierten C₃-C₆-Cycloalkyl-, substituierten oder unsubstituierten fünf- bis neungliedrigen heterocyclischen Gruppe besteht (die 1-3 Heteroatome enthält, welche aus O, S oder N ausgewählt sind);
X aus der Gruppe ausgewählt ist, die aus H und D besteht;
wobei "substituiert" bedeutet, dass ein oder mehrere Wasserstoffatome an einer Gruppe jeweils unabhängig durch einen Substituenten ersetzt sind, der aus der Gruppe ausgewählt ist, die aus Halogen, halogeniertem C₁-C₆-Alkyl, halogeniertem C₁-C₆-Alkoxy, C₁-C₆-Alkyl, C₁-C₆-Alkoxy, C₃-C₆-Cycloalkyl, C₃-C₆-Cycloalkoxy, Cyano und Nitro besteht.

2. Verbindung gemäß Anspruch 1, wobei R¹ aus der Gruppe ausgewählt ist, die aus einem substituierten oder unsubstituierten C₁-C₆-Alkyl und einem substituierten oder unsubstituierten C₃-C₆-Cycloalkyl besteht;
wobei "substituiert" bedeutet, dass ein oder mehrere Wasserstoffatome an einer Gruppe jeweils unabhängig durch einen Substituenten ersetzt sind, der aus der Gruppe ausgewählt ist, die aus Halogen, halogeniertem C₁-C₆-Alkyl, halogeniertem C₁-C₆-Alkoxy, C₁-C₆-Alkyl, C₁-C₆-Alkoxy, C₃-C₆-Cycloalkyl, C₃-C₆-Cycloalkoxy, Cyano und Nitro besteht.

3. Verbindung gemäß Anspruch 1, wobei Ar aus der Gruppe ausgewählt ist, die aus einem substituierten oder unsubstituierten C₆-C₁₀-Aryl, einem substituierten oder unsubstituierten fünf- bis neungliedrigen Heteroarylring besteht;
und der Substituent aus der Gruppe ausgewählt ist, die aus H, F, Cl, Br, Methyl, Ethyl, n-Propyl, Isopropyl, n-Butyl, Isobutyl, Trifluormethyl und Trifluormethoxy besteht.

4. Verbindung gemäß Anspruch 1, wobei A aus der Gruppe ausgewählt ist, die aus einem substituierten oder unsubstituierten C₆-C₁₀-Aryl, einem substituierten oder unsubstituierten fünf- bis neungliedrigen Heteroaryl besteht;
und der Substituent an dem Aryl oder Heteroaryl aus der Gruppe ausgewählt ist, die aus H, F, Cl, Br, Methyl, Ethyl, n-Propyl, Isopropyl, n-Butyl, Isobutyl, Trifluormethyl und Trifluormethoxy besteht.

5. Verbindung gemäß Anspruch 1, wobei R¹ aus der Gruppe ausgewählt ist, die aus Methyl, Ethyl, n-Propyl, Isopropyl, n-Butyl, Isobutyl, Cyclopropyl, Cyclobutyl und Cyclopentyl besteht.

6. Verbindung gemäß Anspruch 1, die aus der Gruppe ausgewählt ist, die aus Folgenden besteht:

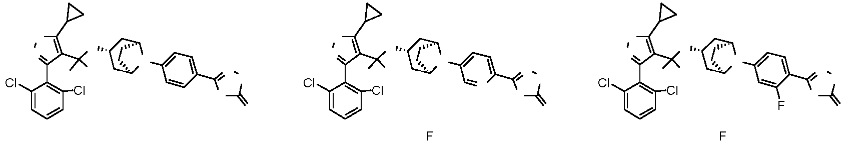

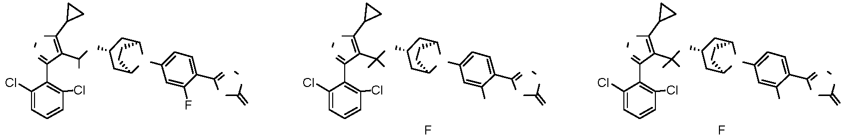

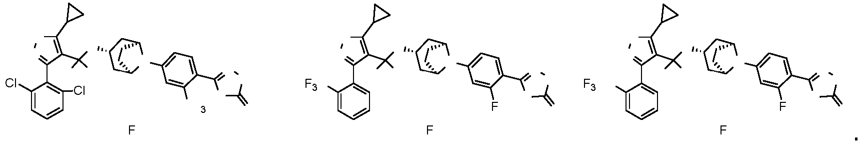

7. Herstellungsverfahren für die Verbindung gemäß Anspruch 1, umfassend das Herstellen einer Verbindung der Formel I durch ein Verfahren, das aus der folgenden Route 1 oder 2 ausgewählt ist: 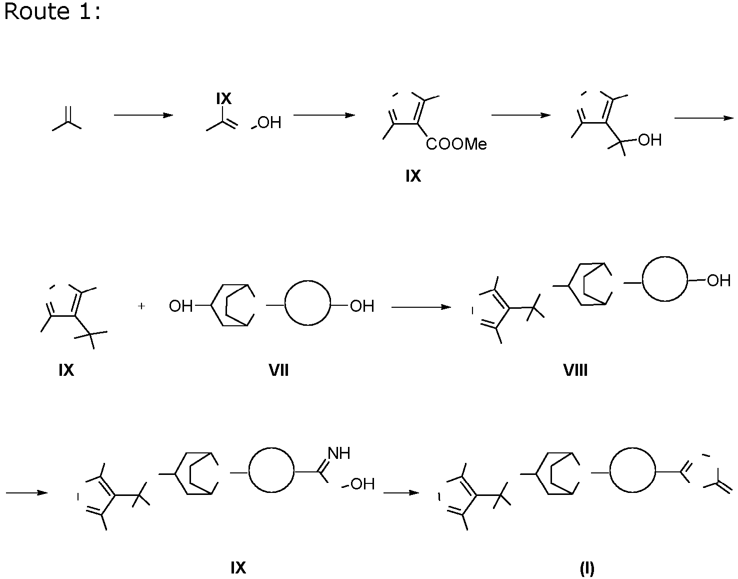
(a) ein substituierter Benzaldehyd der Formel II als Ausgangsstoff wird in Gegenwart einer Base mit Hydroxylamin-Hydrochlorid umgesetzt, wobei man das Zwischenprodukt erhält; das Zwischenprodukt wird dann durch N-Chlorsuccinimid chloriert, wobei eine Verbindung der allgemeinen Formel III entsteht;
(b) die Verbindung der Formel III wird dann in Gegenwart einer Base mit dem entsprechenden 3-Oxopropionat umgesetzt, wobei man eine Verbindung der Formel IV erhält;
(c) die Esterverbindung der Formel IV wird in Gegenwart eines deuterierten Reduktionsmittels zu dem entsprechenden Alkohol, d.h. einer Verbindung der Formel V, reduziert;
(d) die Verbindung der Formel V wird mit einem Bromreagens bromiert, wobei man eine Verbindung der Formel VI erhält;
(e) die Verbindung der Formel VI und die Verbindung der Formel VII werden in Gegenwart einer Base miteinander umgesetzt, wobei man eine Verbindung der Formel VIII erhält;
(f) die Verbindung der Formel VIII wird in Gegenwart einer Base mit Hydroxylamin-Hydrochlorid umgesetzt, wobei man eine Verbindung der Formel IX erhält;
(g) die Verbindung der Formel IX wird in Gegenwart eines Phosgens, Triphosgens oder Carbonyldiimidazols umgesetzt, wobei man die Verbindung der Formel I erhält;
wobei X ein Deuterium ist; und
R¹, Ar, A werden so definiert, wie es in Anspruch 1 beschrieben ist; 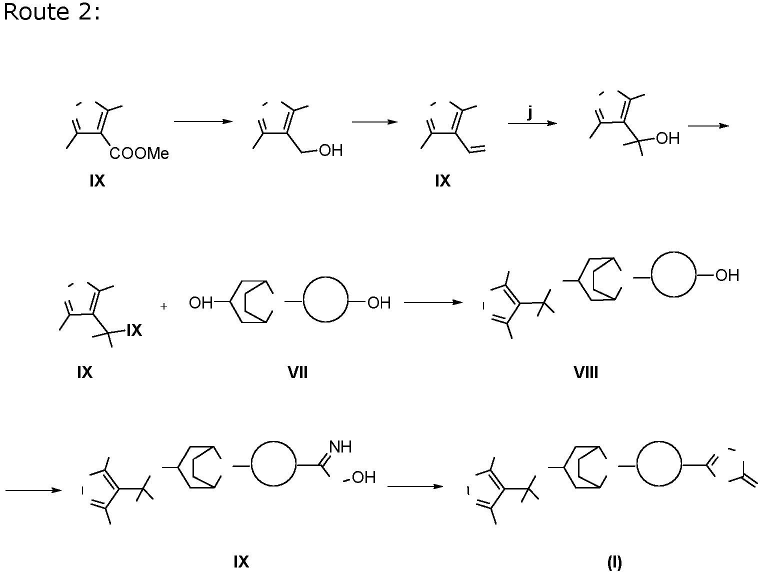
(a) eine Esterverbindung der Formel IV wird in Gegenwart eines Reduktionsmittels zu dem entsprechenden Alkohol, d.h. einer Verbindung der Formel X, reduziert;
(b) die Verbindung der Formel X wird unter der Wirkung eines Oxidationsmittels zu dem entsprechenden Aldehyd, d.h. einer Verbindung der Formel XI, oxidiert;
(c) die Esterverbindung der Formel XI wird in Gegenwart eines deuterierten Reduktionsmittels zu einer Verbindung der Formel V reduziert;
(d) die Verbindung der Formel V wird mit einem Bromreagens bromiert, wobei man eine Verbindung der Formel VI erhält;
(e) die Verbindung der Formel VI und eine Verbindung der Formel VII werden in Gegenwart einer Base miteinander umgesetzt, wobei man eine Verbindung der Formel VIII erhält;
(f) die Verbindung der Formel VIII wird in Gegenwart einer Base mit Hydroxylamin-Hydrochlorid umgesetzt, wobei man eine Verbindung der Formel IX erhält;
(g) die Verbindung der Formel IX wird in Gegenwart eines Phosgens, Triphosgens oder Carbonyldiimidazols umgesetzt, wobei man die Verbindung der Formel I erhält;
wobei X ein Wasserstoff ist; und
R¹, Ar, A werden so definiert, wie es in Anspruch 1 beschrieben ist.

8. Pharmazeutische Zusammensetzung, umfassend die Verbindung der Formel I gemäß Anspruch 1 oder ein Enantiomer, Diastereomer, Tautomer, Racemat, Hydrat, Solvat oder pharmazeutisch annehmbares Salz davon;
und einen pharmazeutisch annehmbaren Träger.

9. Verwendung der Verbindung der Formel I gemäß Anspruch 1 oder eines Enantiomers, Diastereomers, Tautomers, Racemats, Hydrats, Solvats oder eines pharmazeutisch annehmbaren Salzes davon zur Herstellung einer pharmazeutischen Zusammensetzung zur Behandlung von Erkrankungen oder Zuständen, die mit FXR-Aktivität oder -Expression zusammenhängen.

10. Verwendung gemäß Anspruch 9, wobei die mit FXR zusammenhängenden Erkrankungen aus der Gruppe ausgewählt sind, die aus Erkrankungen besteht, welche mit dem Gallensäurestoffwechsel, Glucosestoffwechsel, Lipidstoffwechsel, Entzündung und/oder Leberfibroseprozess zusammenhängen.

## Revendications

1. Composé de formule I : 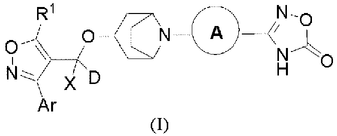
ou énantiomère, diastéréomère, tautomère, racémate, hydrate, solvate ou sel pharmaceutiquement acceptable de celui-ci ;
dans lequel :
Ar est choisi dans le groupe constitué d'un aryle en C₆-C₁₀, substitué ou non substitué, et d'un cycle hétéroaryle à 5 à 9 chaînons, substitué ou non substitué (incluant un cycle simple ou un cycle fusionné, et contenant 1 à 3 hétéroatomes choisis parmi O, S ou N) ;
A est choisi dans le groupe constitué d'un aryle en C₆-C₁₀ substitué ou non substitué, d'un cycle hétéroaryle à 5 à 9 chaînons substitué ou non substitué (incluant un cycle simple ou un cycle fusionné, et contenant 1 à 3 hétéroatomes choisis parmi O, S ou N) ;
R¹ est choisi dans le groupe constitué d'un alkyle en C₁-C₆ substitué ou non substitué, d'un cycloalkyle en C₃-C₆ substitué ou non substitué, d'un groupe hétérocyclique à 5 à 9 chaînons substitué ou non substitué (contenant 1 à 3 hétéroatomes choisis parmi O, S ou N) ;
X est choisi dans le groupe constitué de H et D ;
dans lequel « substitué » signifie qu'un ou plusieurs atomes d'hydrogène sur un groupe sont chacun remplacés indépendamment par un substituant choisi dans le groupe constitué d'un halogène, alkyle en C₁-C₆ halogéné, alcoxy en C₁-C₆ halogéné, alkyle en C₁-C₆, alcoxy en C₁-C₆, cycloalkyle en C₃-C₆, cycloalcoxy en C₃-C₆, cyano et nitro.

2. Composé selon la revendication **1,** dans lequel R¹ est choisi dans le groupe constitué d'un alkyle en C₁-C₆, substitué ou non substitué, et d'un cycloalkyle en C₃-C₆, substitué ou non substitué ;
dans lequel le terme « substitué » signifie qu'un ou plusieurs atomes d'hydrogène sur un groupe sont chacun remplacés indépendamment par un substituant choisi dans le groupe constitué d'un halogène, alkyle en C₁-C₆ halogéné, alcoxy en C₁-C₆ halogéné, alkyle en C₁-C₆, alcoxy en C₃-C₆, cycloalkyle en C₃-C₆, cycloalcoxy en C₃-C₆, cyano et nitro.

3. Composé selon la revendication **1,** dans lequel Ar est choisi dans le groupe constitué d'un aryle en C₆-C₁₀ substitué ou non substitué, d'un cycle hétéroaryle à 5 à 9 chaînons substitué ou non substitué ;
et le substituant est choisi dans le groupe constitué de H, F, CI, Br, méthyle, éthyle, n-propyle, isopropyle, n-butyle, isobutyle, trifluorométhyle et trifluorométhoxy.

4. Composé selon la revendication **1,** dans lequel A est choisi dans le groupe constitué d'un aryle en C₆-C₁₀ substitué ou non substitué, d'un hétéroaryle à 5 à 9 chaînons substitué ou non substitué ;
et le substituant sur l'aryle ou l'hétéroaryle est choisi dans le groupe constitué de H, F, CI, Br, méthyle, éthyle, n-propyle, isopropyle, n-butyle, isobutyle, trifluorométhyle et trifluorométhoxy.

5. Composé selon la revendication **1,** dans lequel R¹ est choisi dans le groupe constitué de méthyle, éthyle, n-propyle, isopropyle, n-butyle, isobutyle, cyclopropyle, cyclobutyle et cyclopentyle.

6. Composé selon la revendication **1,** choisi dans le groupe constitué de

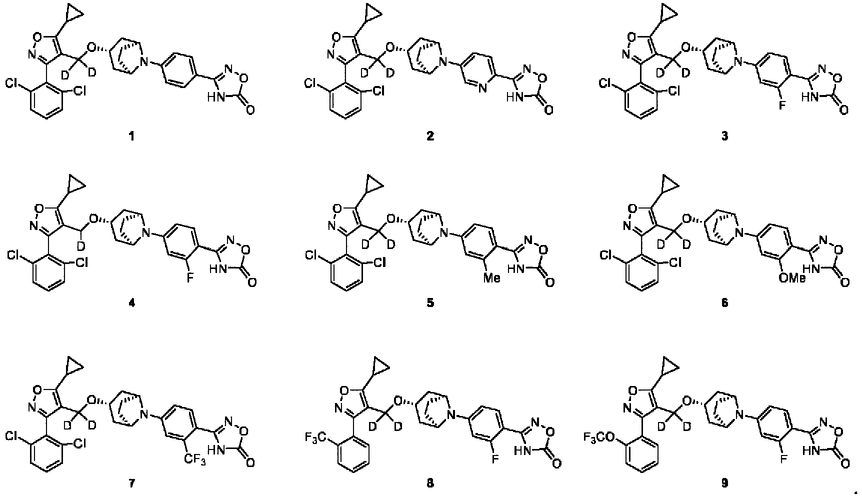

7. Procédé de préparation du composé selon la revendication **1,** comprenant la préparation d'un composé de formule I par un procédé choisi parmi les voies 1 ou 2 suivantes : 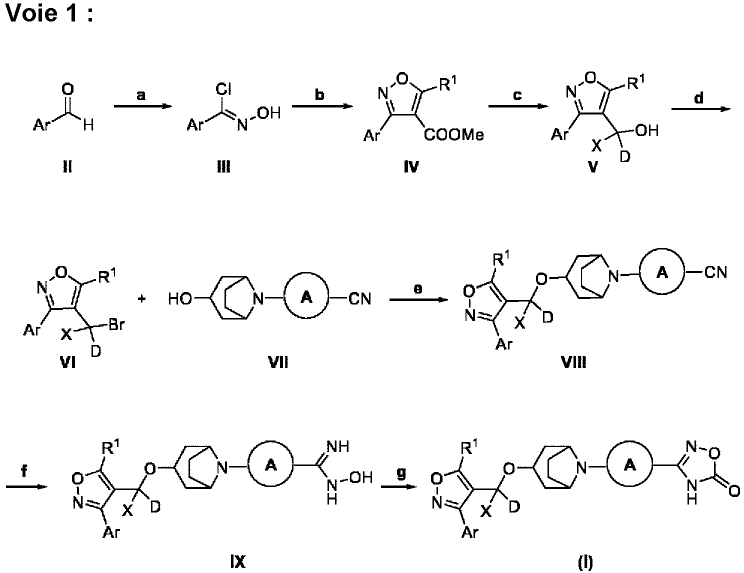
(a) un benzaldéhyde substitué de formule **II,** en tant que matériau de départ, est mis en réaction avec du chlorhydrate d'hydroxylamine en présence d'une base pour obtenir l'intermédiaire ; l'intermédiaire est ensuite chloré par le N-chlorosuccinimide (NCS) pour former un composé de formule générale **III** ;
(b) le composé de formule **III** est ensuite mis en réaction avec le 3-oxopropionate correspondant en présence d'une base pour obtenir un composé de formule **IV** ;
(c) le composé ester de formule **IV** est réduit en alcool correspondant, c'est-à-dire à un composé de formule **V,** en présence d'un agent réducteur deutéré ;
(d) le composé de formule V est bromé par un réactif au brome pour obtenir un composé de formule **VI ;**
(e) le composé de formule **VI** et le composé de formule **VII** sont mis en réaction en présence d'une base pour obtenir un composé de formule **VIII** ;
(f) le composé de formule **VIII** est mis en réaction avec le chlorhydrate d'hydroxylamine en présence d'une base pour obtenir un composé de formule **IX** ;
(g) le composé de formule **IX** est mis en réaction en présence d'un phosgène, triphosgène ou carbonyldiimidazole pour obtenir le composé de formule I ;
où X est un deutérium ; et
R¹, Ar, A sont déterminés selon la revendication 1 ; 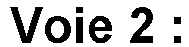 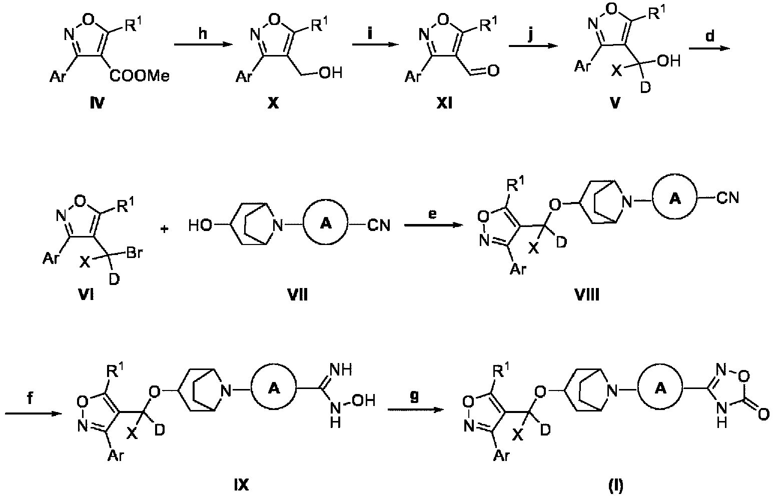
(a) un composé ester de formule **IV** est réduit en alcool correspondant, c'est-à-dire à un composé de formule **X,** en présence d'un agent réducteur ;
(b) le composé de formule **X** est oxydé en aldéhyde correspondant, c'est-à-dire en composé de formule **XI,** sous l'action d'un agent oxydant ;
(c) le composé ester de formule **XI** est réduit en un composé de formule V en présence d'un agent réducteur deutéré ;
(d) le composé de formule **V** est bromé par un réactif au brome pour obtenir un composé de formule **VI ;**
(e) le composé de formule **VI** et un composé de formule **VII** sont mis en réaction en présence d'une base pour obtenir un composé de formule **VIII** ;
(f) le composé de formule **VIII** est mis en réaction avec le chlorhydrate d'hydroxylamine en présence d'une base pour obtenir un composé de formule **IX** ;
(g) le composé de formule **IX** est mis en réaction en présence d'un phosgène, triphosgène ou carbonyldiimidazole pour obtenir le composé de formule **I** ;
où X est un hydrogène ; et
R¹, Ar, A sont déterminés selon la revendication **1.**

8. Composition pharmaceutique comprenant le composé de formule I selon la revendication 1, ou un énantiomère, diastéréomère, tautomère, racémate, hydrate, solvate ou sel pharmaceutiquement acceptable de celui-ci ;
et un support pharmaceutiquement acceptable.

9. Utilisation du composé de formule I selon la revendication **1,** ou d'un énantiomère, diastéréomère, tautomère, racémate, hydrate, solvate ou sel pharmaceutiquement acceptable de celui-ci pour la préparation d'une composition pharmaceutique destinée à traiter des maladies ou des problèmes de santé liés à l'activité ou à l'expression du FXR.

10. Utilisation selon la revendication **9,** dans laquelle les maladies liées au FXR sont choisies dans le groupe constitué de maladies liées au métabolisme des acides biliaires, au métabolisme du glucose, au métabolisme des lipides, à l'inflammation et/ou au processus de fibrose hépatique.
